# EUROPEAN PATENT APPLICATION

(11) **EP 4 596 677 A1**
(43) Date of publication of application: **06.08.2025**
(21) Application number: 23872434.8
(22) Date of filing: 27.09.2023
(51) Int. Cl.: C12N 5/071, A61K 35/12, A61P 1/00, C12Q 1/02, C12Q 1/06, C12N 9/99

(54) **DRUG FOR CULTURING ORGANOID IN ABSENCE OF EXTRACELLULAR MATRIX**

(30) Priority: 27.09.2022 JP 2022153698; 31.05.2023 JP 2023090460
(71) Applicant: Keio University, Tokyo, 108-8345 (JP)
(72) Inventor: SATO Toshiro, Tokyo 160-8582 (JP); YANO Tomoki, Tokyo 160-8582 (JP)
(74) Representative: Cabinet Becker et Associés
(86) International application number: PCT/JP2023/035181
(87) International publication number: WO 2024/071212

(57) **Abstract**

Provided are: an agent for organoid formation and proliferation in the absence of an extracellular matrix, the agent including a Hippo signaling pathway inhibitor as an active component; a method for organoid proliferation; an organoid proliferated by the above-described proliferation method; an agent for establishing an organoid in the absence of an extracellular matrix; a method for producing an organoid; an organoid produced by the above-described production method; a regenerative medicine preparation; and a method for screening for an agent that enables culture of an organoid in the absence of an extracellular matrix.

## Description

### TECHNICAL FIELD

The present invention relates to an agent for culturing an organoid in the absence of an extracellular matrix. In more detail, the present invention relates to an agent for organoid formation and proliferation in the absence of an extracellular matrix, a method for organoid proliferation, an organoid proliferated by the above-described proliferation method, an agent for establishing an organoid in the absence of an extracellular matrix, a method for producing an organoid, an organoid produced by the above-described production method, a regenerative medicine preparation, and a method for screening for an agent that enables culture of an organoid in the absence of an extracellular matrix. Priority is claimed on Japanese Patent Application No. 2022-153698, filed on September 27, 2022 and Japanese Patent Application No. 2023-090460, filed on May 31, 2023, the contents of which are incorporated herein by reference.

### BACKGROUND ART

Organoid culture technology has been developed and is expected to be applied to drug discovery and regenerative medicine. Extracellular matrices such as Matrigel (registered trademark) are required to culture organoids (for example, refer to Patent Document 1, Non-Patent Document 1, and the like). Matrigel (registered trademark) is an animal-derived product because it is an extract from a tumor implanted in mice. In addition, use of Matrigel (registered trademark) in culture of organoids is a barrier to medical applications due to the possibility of contamination with unknown viruses and its high cost. Furthermore, the use of Matrigel has become a challenge from an animal welfare perspective. Since the advent of organoid technology, there has been vigorous development of substitutes for Matrigel (registered trademark) (for example, refer to Non Patent Document 2).

### Citation List

### Patent Document

Patent Document 1: Japanese Unexamined Patent Application, First Publication No. 2016-198033

### Non Patent Documents

Non-Patent Document 1: S Rezakhani, et al., Extracellular matrix requirements for gastrointestinal organoid cultures, Biomaterials, 276, 121020, 2021
Non-Patent Document 2: Jeong Hyun Heo, et al., Engineering the Extracellular Matrix for Organoid Culture, Int J Stem Cells, 15 (1), 60-69, 2022

### SUMMARY OF INVENTION

### Technical Problem

Conventionally, synthetic extracellular matrices, collagen, and the like are sometimes used as alternatives for Matrigel (registered trademark), but they have not achieved sufficient organoid culture efficiency and have not been widely adopted.

Therefore, an object of the present invention is to provide technology for producing and culturing an organoid in the absence of an extracellular matrix.

### Solution to Problem

The present invention has the following aspects.
[1] An agent for organoid formation and proliferation in the absence of an extracellular matrix, the agent including: a Hippo signaling pathway inhibitor as an active component.
[2] The agent for organoid formation and proliferation in the absence of an extracellular matrix according to [1], in which the Hippo signaling pathway inhibitor is an MST1/2 kinase inhibitor or a large tumor suppressor kinase (LATS) 1/2 kinase inhibitor.
[3] The agent for organoid formation and proliferation in the absence of an extracellular matrix according to [1] or [2], the agent further including: a cytokine family that binds to gp130.
[4] The agent for organoid formation and proliferation in the absence of an extracellular matrix according to any one of [1] to [3], in which the organoid is an epithelial organoid.
[5] A method for organoid proliferation, including: a step of culturing an organoid in the presence of a Hippo signaling pathway inhibitor and in the absence of an extracellular matrix.
[6] The method for organoid proliferation according to [5], in which the organoid is cultured under serum-free conditions.
[7] An organoid proliferated by the proliferation method according to [5] or [6].
[8] An agent for establishing an organoid in the absence of an extracellular matrix, the agent including: a Hippo signaling pathway inhibitor as an active component.
[9] The agent for establishing an organoid in the absence of an extracellular matrix according to [8], in which the Hippo signaling pathway inhibitor is an MST1/2 kinase inhibitor or a LATS1/2 kinase inhibitor.
[10] The agent for establishing an organoid in the absence of an extracellular matrix according to [8] or [9], the agent further including: a cytokine family that binds to gp 130.
[11] The agent for establishing an organoid in the absence of an extracellular matrix according to any one of [8] to [10], in which the organoid is an epithelial organoid.
[12] A method for producing an organoid, including: a step of establishing an organoid in the presence of a Hippo signaling pathway inhibitor and in the absence of an extracellular matrix.
[13] The method for producing an organoid according to [12], in which the organoid is established in the presence of a cytokine family that binds to gp130.
[14] The method for producing an organoid according to [12] or [13], in which the organoid is established under serum-free conditions.
[15] An organoid produced by the production method according to any one of [12] to [14].
[16] A regenerative medicine preparation including: the organoid according to [15] as an active component.
[17] A method for screening for an agent that enables culture of an organoid in the absence of an extracellular matrix, the method including: a step of culturing an organoid in the presence of a test substance and in the absence of an extracellular matrix; and a step of evaluating proliferation of the organoid, in which an increase in the proliferation of the organoid compared to when the test substance is absent indicates that the test substance is an agent that enables organoid culture in the absence of an extracellular matrix.

### Advantageous Effects of Invention

According to the present invention, it is possible to provide technology for culturing an organoid in the absence of an extracellular matrix.

### BRIEF DESCRIPTION OF DRAWINGS

[FIG. 1] A schematic view explaining the Hippo signaling pathway.
[FIG. 2] Representative photographs and graphs showing the results of Experimental Example 1.
[FIG. 3] Representative photographs and graphs showing the results of Experimental Example 2.
[FIG. 4] Representative photographs showing the results of Western blotting in Experimental Example 3.
[FIG. 5] Representative photographs showing the results of immunostaining of human small intestine-derived organoids in Experimental Example 4.
[FIG. 6] Representative photographs showing the results of immunostaining of human large intestine-derived organoids in Experimental Example 4.
[FIG. 7] A graph summarizing the results of FIGS. 5 and 6.
[FIG. 8] A graph showing the results of Experimental Example 5.
[FIG. 9] Representative photographs showing the results of Experimental Example 6.
[FIG. 10] A graph showing the results of Experimental Example 7.
[FIG. 11] Representative photographs showing the results of Experimental Example 8.
[FIG. 12] Representative photographs showing the results of immunostaining of organoids in Experimental Example 9.
[FIG. 13] Representative photographs showing the results of immunostaining of organoids in Experimental Example 10.
[FIG. 14] Graphs showing the results of Experimental Example 11.
[FIG. 15] Graphs showing the results of Experimental Example 11.
[FIG. 16] Graphs showing the results of quantitative real-time PCR in Experimental Example 12.
[FIG. 17] Graphs showing the results of Western blotting in Experimental Example 12.
[FIG. 18] Representative photographs showing the results of immunostaining of organoids in Experimental Example 13.
[FIG. 19] Representative micrographs of organoids in Experimental Example 13.
[FIG. 20] Representative photographs showing the results of immunostaining of organoids in Experimental Example 14.
[FIG. 21] Representative micrographs of organoids in Experimental Example 14.
[FIG. 22] A graph showing the results of Experimental Example 15.
[FIG. 23] Representative micrographs of organoids in Experimental Example 16.
[FIG. 24] Graphs showing the results of Experimental Example 16.
[FIG. 25] Representative photographs showing the results of immunostaining of organoids in Experimental Example 17.
[FIG. 26] Graphs showing the results of Experimental Example 18.
[FIG. 27] A graph showing the results of Experimental Example 19.
[FIG. 28] A graph showing the results of Experimental Example 20.
[FIG. 29] Micrographs and graphs showing the results of Experimental Example 21.
[FIG. 30] A schematic view explaining an overview of Experimental Example 22 and a graph showing the results of Experimental Example 22.
[FIG. 31] A schematic view explaining an overview of Experimental Example 23.
[FIG. 32] Photographs showing the results of Experimental Example 23.

### DESCRIPTION OF EMBODIMENTS

### [Agent for proliferation of organoid in absence of extracellular matrix]

In one embodiment, the present invention provides an agent for organoid formation and proliferation in the absence of an extracellular matrix, the agent including a Hippo signaling pathway inhibitor as an active component.

As will be described below in examples, addition of the agent of the present embodiment to a medium enables formation and proliferation of an organoid in the absence of an extracellular matrix. Here, "proliferation" means an increase in the number of cells constituting an organoid and can also be rephrased as "culture." In addition, "to form" an organoid refers to, for example, establishing an organoid and re-forming an organoid from an organoid that has been dissociated into a single cell. In one aspect, the agent of the present embodiment may be an agent for proliferation of an organoid in the absence of an extracellular matrix which contains a Hippo signaling pathway inhibitor as an active component.

Conventionally, organoid proliferation was not possible in the absence of an extracellular matrix. The agent of the present embodiment enables organoid proliferation without using any animal-derived products such as Matrigel (registered trademark), which is an extracellular matrix. Here, animal-derived products are substances derived from human or non-human animals with unidentified components. It is expected that organoids will be applied to regenerative medicine through culture technology free of animal-derived products. In addition, it is more advantageous than conventional organoid culture technology in terms of animal welfare and cost.

The Hippo signaling pathway is a signaling pathway known to be involved in cell proliferation, apoptosis, and stem cell self-renewal, and is known to be an evolutionarily conserved pathway.

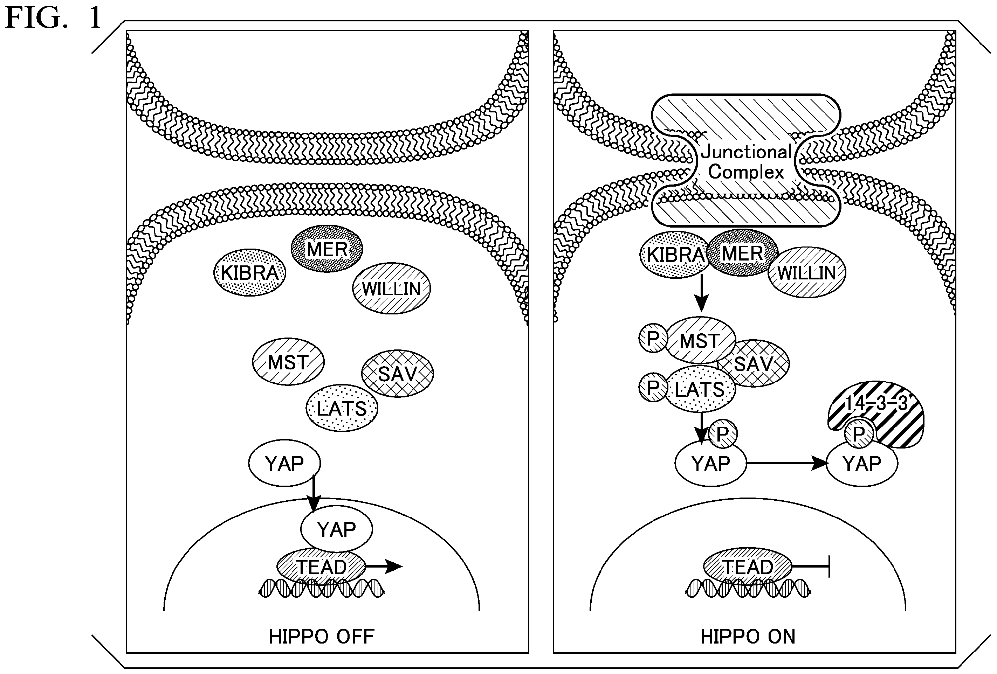

FIG. 1 is a schematic view explaining the Hippo signaling pathway. As shown in FIG. 1, the transcription factor TEAD is known to activate transcription of genes involved in cell proliferation and promote cell proliferation by binding to Yes-associated protein (YAP), a coactivator. YAP exists in both a phosphorylated form and a non-phosphoric acid form. Non-phosphoric acid form YAP translocates into the nucleus and acts as a transcriptional coactivator of TEAD. On the other hand, phosphoric acid form YAP binds to cytoplasmic protein 14-3-3 and cannot translocate into the nucleus, and therefore cannot act as a coactivator. Therefore, phosphorylation regulation, which determines nuclear translocation of YAP, is a very important event in cell proliferation.

In addition, as shown in FIG. 1, large tumor suppressor kinase (LATS) is known to negatively regulate YAP's involvement in cell proliferation by phosphorylating YAP and localizing YAP to the cytoplasm.

Hippo signaling pathway inhibitors are not particularly limited as long as they inhibit any of the above-described signaling pathway steps.

For example, they may be MST1 kinase or MST2 kinase inhibitors (MST1/2 kinase inhibitors), or may be LATS1 kinase or LATS2 kinase inhibitors (LATS 1/2 kinase inhibitors).

NCBI accession numbers of the amino acid sequences of human MST1 kinase are NP_001380510.1, NP_001380511.1, NP_001380512.1, NP_001380513.1, NP_001380514.1, NP_066278.3, and the like. In addition, NCBI accession numbers of the amino acid sequences of human MST2 kinase are NP_001243241.1, NP_001243242.1, NP_006272.2, and the like.

NCBI accession numbers of the amino acid sequences of human LATS1 kinase are NP_001257448.1, NP_001337268.1, NP_001337269.1, NP_001337321.1, NP_004681.1, and the like. **In** addition, an NCBI accession number of the amino acid sequence of human LATS2 kinase is NP_055387.2, or the like.

More specific examples of Hippo signaling pathway inhibitors may include inhibitors of LATS1 kinase and LATS2 kinase, Lats-IN-1 (CAS No. 1424635-83-5, hereinafter sometimes referred to as "Lats-IN" and also referred to as "TRULI"), GA-017 (CAS No. 2351906-74-4), and TDI-011536 (CAS No. 2687970-96-1).

Examples of extracellular matrices include Matrigel (registered trademark), collagen, fibronectin, proteoglycans, and laminin. Conventionally, organoid proliferation was not possible in the absence of an extracellular matrix.

Proliferation of an organoid in the absence of an extracellular matrix means that no extracellular matrix is added externally to a medium of the organoid, and it is acceptable for trace amounts of extracellular matrix produced by the organoid itself to be incorporated into a medium, or for trace amounts of extracellular matrix to be unintentionally incorporated into a medium. Here, trace amounts may be at a level around the detection limit.

Examples of organoids in the agent of the present embodiment include epithelial organoids, such as small intestine-derived organoids, large intestine-derived organoids, stomach-derived organoids, biliary tract-derived organoids, pancreatic organoids, mammary gland-derived organoids, liver cell organoids, lung organoids, airway organoids, esophageal organoids, and salivary gland organoids.

Organoids may be human-derived organoids or non-human animal-derived organoids. Examples of non-human animals include mammals, such as rodents such as mice, rats, hamsters, and guinea pigs; hoofed animals such as pigs, cattle, goats, horses, and sheep; carnivores such as dogs and cats; and primates such as rhesus macaques, crab-eating macaques, marmosets, orangutans, and chimpanzees.

The agent of the present embodiment is added to an organoid culture medium and used. The concentration of the agent of the present embodiment in the organoid culture medium is preferably about 10 µM to 60 µM and more preferably about 10 µM to 30 µM.

Examples of organoid culture media include one obtained by removing an extracellular matrix from a conventional organoid culture medium. As the organoid culture medium, a medium obtained by adding at least one of the following components i) to v) to a basal medium is preferable. The organoid culture medium can also contain serum, but it is preferred to be serum-free from the viewpoint of being free of animal-derived products.
i) Wnt Agonist
ii) At least one selected from the group consisting of insulin-like growth factor 1 (IGF1), fibroblast growth factor 2 (FGF2), epidermal growth factor (EGF), epiregulin (EREG), fibroblast growth factor 10 (FGF10), and gastrin-1
iii) Bone morphogenetic protein (BMP) inhibitor
iv) Transforming growth factor-β (TGF-β) inhibitor
v) γ-Secretase inhibitor

The organoid culture medium preferably contains at least one selected from the group consisting of IGF1, FGF2, EGF, and epiregulin. Which of IGF1, FGF2, EGF, and epiregulin is contained can be selected as appropriate. The organoid culture medium preferably contains IGF1 and epiregulin, FGF2 and epiregulin, or IGF1 and FGF2, and more preferably contains IGF1 and FGF2.

Any serum-free basal cell culture medium can be used as the basal medium. Examples thereof include a defined synthetic medium that is buffered with a carbonate-based buffer at a pH of 7.2 to 7.6. More specific examples thereof include glutamine, insulin, B27 supplement (Thermo Fisher Scientific Inc.), N-acetyl-L-cysteine (FUJIFILM Wako Pure Chemical Corporation), penicillin, streptomycin, Advanced Dulbecco's Modified Eagle's Medium/Ham's F-12 mixed medium (DMEM/F12) supplemented with transferrin or the like. In addition, RPMI 1640 medium, Advanced RPMI medium, or the like may be used instead of DMEM/F12 medium.

### (Wnt Agonist)

The Wnt agonist means an agent that activates T-cell factor (hereinafter also referred to as TCF)/lymphoid enhancer factor (hereinafter referred to as LEF)-mediated transcription within cells. Accordingly, the Wnt agonist is not limited to a Wnt family protein, and includes a Wnt agonist that binds to and activates a Frizzled receptor family member, an inhibitor of intracellular β-catenin degradation, and an activator of TCF/LEF. The Wnt agonist is preferably at least one selected from the group consisting of Wnt protein, R-spondin, and a GSK-3β inhibitor.

The organoid culture medium preferably contains a Wnt agonist. A Wnt agonist more preferably includes a complex of Wnt protein and afamin, a stabilizer thereof, and still more preferably includes a complex of Wnt protein and afamin, and R-spondin.

### <<Wnt Protein>>

The origin of Wnt protein is not particularly limited, and Wnt proteins derived from various organisms can be used. Of these, Wnt proteins derived from mammals are preferable. Examples of mammals include ones similar to those described above. Examples of mammalian Wnt proteins include Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16. In the organoid culture medium, multiple Wnt proteins may be used in combination.

Examples of methods for preparing Wnt protein include a preparation method using Wnt protein-expressing cells. The origin (such as organism species and culture forms) of the Wnt protein-expressing cells is not particularly limited. Any cells that stably express Wnt protein may be used, and any cells that transiently express Wnt protein may be used. Examples of Wnt protein-expressing cells include L cells that stably express mouse Wnt3a (ATCC CRL-2647) and L cells that stably express mouse Wnt5a (ATCC CRL-2814). In addition, Wnt protein-expressing cells can be produced using a well-known gene recombination technique. In other words, by inserting DNA encoding desired Wnt protein into well-known expression vectors and introducing the resulting expression vectors into appropriate host cells, Wnt protein-expressing cells can be produced. Nucleotide sequences of genes encoding desired Wnt protein can be acquired from a well-known database such as GenBank, for example.

Wnt protein expressed by Wnt protein-expressing cells may be a fragment of the Wnt protein as long as it has Wnt activity, and may contain amino acid sequences other than the amino acid sequence of the Wnt protein. The amino acid sequences other than the amino acid sequence of the Wnt protein are not particularly limited, and examples thereof include amino acid sequences of affinity tags. In addition, it is unnecessary for the amino acid sequence of the Wnt protein to completely coincide with amino acid sequences that can be acquired from a well-known database such as GenBank, and may be substantially the same amino acid sequences as the amino acid sequences that can be acquired from a well-known database as long as it has Wnt activity.

Examples of the amino acid sequences substantially the same as the amino acid sequence of the Wnt protein that can be acquired from a well-known database such as GenBank include an amino acid sequence in which one to several amino acids are deleted, substituted, or added in the amino acid sequences that can be acquired from a well-known database.

The amino acid sequence in which one to several amino acids are deleted, substituted, or added means an amino acid sequence in which a sufficient number of amino acids (10 or less is preferable, 7 or less is more preferable, and 6 or less is still more preferable) are deleted, substituted, or added to enable deletion, substitution, or addition through a well-known mutant peptide production method such as site-specific mutation induction method.

In addition, examples of substantially the same amino acid sequences include amino acid sequences having at least 80% or more, preferably at least 85% or more, more preferably at least 90% or more, still more preferably at least 92% or more, particularly preferably at least 95% or more, most preferably at least 99% or more identity to the amino acid sequences that can be acquired from a well-known database.

The concentration of the Wnt protein is preferably 50 ng/mL or more, more preferably 100 ng/mL to 10 µg/mL, still more preferably 200 ng/mL to 1 µg/mL, and particularly preferably 300 ng/mL to 1 µg/mL.

### <<R-Spondin>>

Examples of R-spondin include an R-spondin family consisting of R-spondin 1, R-spondin 2, R-spondin 3, and R-spondin 4. The R-spondin family is a secretory protein known to be involved in activation and regulation of Wnt signaling pathways. In the organoid culture medium, multiple R-spondins may be used in combination. In addition, they may be fragments of R-spondins as long as they have R-spondin activity, and may contain amino acid sequences other than the amino acid sequence of R-spondin.

### <<GSK-3β Inhibitor>>

Examples of GSK-3β inhibitors include, CHIR-99021 (CAS No. 252917-06-9), CHIR-98014 (CAS No. 252935-94-7), lithium, Kenpaullone (CAS No. 142273-20-9), 6-bromoindirubin-30-acetoxime, SB216763 (CAS No. 280744-09-4), SB415286 (CAS No. 264218-23-7), FRAT family members that block an interaction between GSK-3 and axin, and FRAT-derived peptides.

### <<Afamin>>

Afamin means a glycoprotein belonging to the albumin family and is known to be present in blood or body fluids. Serum normally added to a medium contains afamin derived from an animal from which the serum was collected. Since serum contains impurities and the like other than afamin, it is preferable to use afamin alone without using serum.

The afamin contained in the organoid culture medium is not particularly limited in origin, and afamin derived from various organisms can be used. Of these, it is preferable to use afamin derived from mammals. Examples of mammals include ones similar to those described above. The amino acid sequences of afamin from major mammals and the nucleotide sequences of the genes encoding them can be obtained from well-known databases such as GenBank, for example. For example, in GenBank, the amino acid sequence of human afamin is AAA21612, and the nucleotide sequence of the gene encoding this is registered under the accession number L32140. The amino acid sequence of bovine afamin is DAA28569, and the nucleotide sequence of the gene encoding this is registered under the accession number GJ060968.

The afamin contained in the organoid culture medium may be one obtained by purifying natural afamin contained in serum or the like through a well-known method, or may be recombinant afamin. Recombinant afamin can be produced using well-known genetic recombination technology as appropriate.

As a method for producing recombinant afamin, recombinant afamin can be produced by, for example, inserting DNA encoding afamin into a well-known expression vector, introducing the resulting expression vector into appropriate host cells to express recombinant afamin, and performing purification using a well-known purification method. Recombinant may be afamin with an affinity tag added. The affinity tag added is not particularly limited and can be appropriately selected from well-known affinity tags and used. The affinity tag is preferably an affinity tag recognized by a specific antibody, and examples thereof include a FLAG tab, an MYC tag, a HA tag, and a V5 tag.

The above-described Wnt protein is strongly hydrophobic because a specific serine residue is modified with a fatty acid (palmitoleic acid). Therefore, Wnt protein is prone to aggregation or denaturation in an aqueous solution, making its purification and storage particularly challenging, as widely known.

On the other hand, modification of this particular serine residue by a fatty acid is essential for the bioactivity of the Wnt protein and has been reported to be involved in binding to Frizzled receptor family members.

It is also known that in an aqueous solution, Wnt protein binds and forms a complex with afamin on a one-to-one basis, and is solubilized while maintaining high physiological activity. Wnt protein-afamin complexes may be produced by a method for culturing cells expressing both Wnt protein and afamin or by a method for co-culturing Wnt protein-expressing cells and afamin-expressing cells.

The concentration of the afamin contained in the organoid culture medium is not particularly limited, but is preferably 50 ng/mL to 10 µg/mL, more preferably 100 ng/mL to 1 µg/mL, and still more preferably 300 µg/mL to 1 µg/mL.

### (IGF1)

IGF1, also called somatomedin C, is a factor secreted mainly in the liver in response to stimulation by growth hormone (GH). It is known that most cells (particularly cells of muscle, bone, the liver, the kidneys, nerves, the skin the lungs, and the like) in the human body are affected by IGF1. In addition to its insulin-like effects, IGF1 functions to regulate cell proliferation (particularly neurons), development, and cellular DNA synthesis.

The concentration of IGF1 contained in the organoid culture medium is not particularly limited, but is preferably 5 ng/mL to 1 µg/mL, more preferably 10 ng/mL to 1 µg/mL, and still more preferably 50 ng/mL to 500 ng/mL.

### (FGF2)

FGF2 is a basic fibroblast growth factor that binds to the fibroblast growth factor receptor (FGFR) and has the function of promoting proliferation of vascular endothelial cells and their organization into tubular structures, that is, promoting angiogenesis. Human FGF2 is also known to have two isoforms, a low molecular weight form (LWL) and a high molecular weight form (HWL). LWL is present mainly in the cytoplasm and acts in an autocrine manner, whereas HWL is located in the nucleus and is active via an intracrine mechanism acting intracellularly.

The concentration of FGF2 contained in the organoid culture medium is not particularly limited, but is preferably 5 ng/mL to 500 µg/mL, more preferably 10 ng/mL to 300 µg/mL, and still more preferably 50 ng/mL to 100 µg/mL.

### (FGF10)

FGF10 is a molecule which is mainly secreted from mesenchymal tissues and acts on epithelia via FGFR2b, and is responsible for important epithelial-mesenchymal interactions in the formation and repair of various tissues.

The concentration of FGF10 contained in the organoid culture medium is not particularly limited, but is preferably 5 ng/mL to 1 µg/mL, more preferably 10 ng/mL to 1 µg/mL, and still more preferably 30 ng/mL to 500 ng/mL.

### (EGF)

EGF is a potent mitogen for various cultured ectodermal and mesodermal cells and has a significant effect on the differentiation of specific cells of a subset of fibroblasts. An EGF precursor exists as a membrane-bound molecule that is proteolytically cleaved to generate a 53-amino acid peptide hormone that stimulates cells.

The concentration of EGF contained in the organoid culture medium is preferably 5 ng/mL to 500 ng/mL, more preferably 10 ng/mL to 400 ng/mL, and still more preferably 50 ng/mL to 200 ng/mL.

### (EREG)

EREG is an EGF-like growth factor that specifically binds to ErbB1 and ErbB4 among the tyrosine kinase (ErbB) family receptors (ErbB1 to 4). It is known to stimulate proliferation of keratin-producing cells, liver cells, fibroblasts, and vascular endothelial cells. In addition, EREG is expressed mainly in malignant tumors of the bladder, lung, kidney, large intestine, and the like, the placenta, and peripheral blood leukocytes.

The concentration of EREG contained in an organoid culture medium is not particularly limited, but is preferably 5 ng/mL to 1 µg/mL, more preferably 10 ng/mL to 1 µg/mL, and still more preferably 50 ng/mL to 500 ng/mL.

### (BMP inhibitor)

BMPs bind as dimer ligands to a receptor complex consisting of two different receptor serine/threonine kinases, type I and type II receptors. The type II receptor phosphorylates the type I receptor, resulting in activation of the receptor kinase. The type I receptor subsequently phosphorylates a specific receptor substrate (SMAD), which in turn leads to transcriptional activation through the signaling pathway. Generally, a BMP inhibitor is an agent that, for example, blocks or inhibits binding of a BMP molecule to a BMP receptor and binds to the BMP molecule to form a complex that neutralizes BMP activity. In addition, the BMP inhibitor is, for example, an agent that binds to a BMP receptor, blocks or inhibits the binding of a BMP molecule to the receptor, and acts as an antagonist or an inverse agonist.

The BMP inhibitor preferably has an inhibitory activity of 50% or more, more preferably 70% or more, still more preferably 80% or more, and particularly preferably 90% or more, compared to the BMP activity level in the absence of the inhibitor.

The BMP inhibitor is preferably a natural BMP-binding protein, for example, Gremlin, Chordin, and Chordin-like proteins such as Noggin and Chordin domains; Follistatin-related proteins such as Follistatin and Follistatin domains; DAN-like proteins such as DAN and DAN cysteine-knot domains; sclerostin/SOST, decorin, and α-2 macroglobulin.

Among these, as the BMP inhibitor contained in the organoid culture medium, Chordin-like proteins or DAN-like proteins are preferable, and Chordin-like proteins are more preferable. As a Chordin-like protein, Noggin is preferable. Chordin-like proteins and DAN-like proteins are diffusible proteins that bind to BMP molecules with varying degrees of affinity and can inhibit access of the BMP molecules to signaling receptors. When culturing epithelial stem cells, loss of stem cells can be prevented by adding these BMP inhibitors to the organoid culture medium.

The concentration of a BMP inhibitor contained in the organoid culture medium is preferably 10 ng/mL to 100 ng/mL, more preferably 20 ng/mL to 100 ng/mL, and still more preferably 50 ng/mL to 100 ng/mL.

### (TGF-β Inhibitor)

TGF-β is a type of growth factor that is produced by almost all cells of the kidneys, bone marrow, and platelets. There are five subtypes of TGF-β (β1 to β5). In addition, TGF-β is known to promote proliferation of osteoblasts, as well as synthesis and proliferation of connective tissue such as collagen, and to have an inhibitory effect on the proliferation of epithelial cells and osteoclasts. In general, a TGF-β inhibitor is, for example, an agent that blocks or inhibits binding of TGF-β to a TGF-β receptor and binds to TGF-β to form a complex that neutralizes TGF-β activity. In addition, the TGF-β inhibitor is, for example, an agent that binds to a TGF-β receptor, blocks or inhibits the binding of TGF-β to the receptor, and acts as an antagonist or an inverse agonist.

Examples of TGF-β inhibitors include A83-01 (CAS No. 909910-43-6), ALK5 Inhibitor I (3-(pyridine-2-yl)-4-(4-quinonyl)-1H- pyrazole), LDN193189 (CAS No. 1062368-24-4), SB-431542 (CAS No. 301836-41-9), SB-505124 (CAS No. 694433-59-5), SD-208 (CAS No. 627536-09-8), SB-525334 (CAS No. 356559-20-1), LY364947 (CAS No. 396129-53-6), LY2157299 (CAS No. 700874-72-2), TGF-β RI Kinase Inhibitor II 616452 (CAS No. 446859-33-2), TGF-β RI Kinase Inhibitor III 616453 (CAS No. 356559-13-2), TGF-β RI Kinase Inhibitor IX 616463 (4-((4-((2,6-dimethylpyridine-3-yl)oxy)pyridine-2-yl)amino)benzenesulfonamide), TGF-β RI Kinase Inhibitor VII 616458 (CAS No. 666729-57-3), TGF-β RI Kinase Inhibitor VIII 616459 (CAS No. 356559-20-1), AP12009 (TGF-β2 antisense compound "Trabedersen"), belagenpumatucel-L (TGF-β2 antisense gene-modified allogeneic tumor cell vaccine), CAT-152 (Glaucoma-lerdelimumab (anti-TGF-β2 monoclonal antibody)), CAT-192 (metelimumab (a human IgG4 monoclonal antibody that neutralizes TGFβ1), and GC-1008 (anti-TGF-β monoclonal antibody). Of these, A83-01 is preferable as a TGF-β inhibitor.

The concentration of the TGF-β inhibitor contained in the organoid culture medium is preferably 100 nM to 10 µM, more preferably 500 nM to 5 µM, and still more preferably 500 nM or to 2 µM.

### (y-Secretase inhibitor)

Examples of γ-secretase include BMS299897 (CAS No. 290315-45-6), DAPT (CAS No. 208255-80-5), DBZ (CAS No. 209984-56-5), JLK6 (CAS No. 62252-26-0), L-685458 (CAS No. 292632-98-5), and LY411575 (CAS No. 209984-57-6). Of these, LY411575 is preferable.

The concentration of the γ-secretase inhibitor contained in the organoid culture medium is preferably about 1 µM to 10 µM and more preferably about 100 µM to 1 µM.

### (Other components)

The organoid culture medium may further contain a Rho-kinase (ROCK) inhibitor. Examples of ROCK inhibitors include Y-27632 (CAS No. 129830-38-2), fasudil (HA1077) (CAS No. 103745-39-7), and H-1152 (CAS No. 871543-07-6). When Y-27632 is used as a ROCK inhibitor, it is preferable to add it for about the first 2 days of culturing stem cells dispersed into single cells. Y-27632 contained in the organoid culture medium is preferably about 10 µm.

When organoids are liver cell organoids, the organoid culture medium preferably further contains a cytokine of a cytokine family that binds to gp130, such as Oncostatin M. The concentration of Oncostatin M contained in the organoid culture medium may be, for example, 1 ng/mL to 10 µg/mL, 1 ng/mL to 1 µg/mL, or 5 ng/mL to 100 ng/mL.

The organoid culture medium may further be supplemented with gastrin (or a suitable alternative such as Leu15-gastrin). The concentration of gastrin or a suitable substitute contained in the organoid culture medium may be, for example, 1 ng/mL to 10 µg/mL, 1 ng/mL to 1 µg/mL, or 5 ng/mL to 100 ng/mL.

The organoid culture medium may further contain at least one type of amino acid. Examples of amino acids include L-alanine, L-arginine, L-asparagine, L-aspartic acid, L-cysteine, L-cystine, L-glutamic acid, L-glutamine, L-glycine, L-histidine, L-isoleucine, L-leucine, L-lysine, L-methionine, L-phenylalanine, L-proline, L-serine, L-threonine, L-tryptophan, L-tyrosine, L-valine, and combinations thereof. The concentration of L-glutamine contained in the organoid culture medium is 0.05 g/L to 1 g/L (in general, 0.1 g/L to 0.75 g/L). The concentration of other amino acids contained in the organoid culture medium is 0.001 g/L or more and 1 g/L (in general, 0.01 g/L to 0.15 g/L). The amino acids may be synthetic.

The organoid culture medium may further contain at least one type of vitamin. Examples of vitamins include thiamine (vitamin B1), riboflavin (vitamin B2), niacin (vitamin B3), D-calcium pantothenate (vitamin B5), pyridoxal/pyridoxamine/pyridoxine (vitamin B6), folic acid (vitamin B9), cyanocobalamin (vitamin B12), ascorbic acid (vitamin C), calciferol (vitamin D2), DL-α-tocopherol (vitamin E), biotin (vitamin H), and menadione (vitamin K).

The organoid culture medium may further contain at least one type of inorganic salt. Inorganic salts are used to help maintain cellular osmotic balance and to help regulate membrane potential. Specific examples of inorganic salts include salts of calcium, copper, iron, magnesium, potassium, sodium, and zinc. The salts are usually used in the form of chlorides, phosphates, sulfates, nitrates and bicarbonates. More specific examples of salts include CaCl₂, CuSO₄·5H₂O, Fe(NO₃)·9H₂O, FeSO₄·7H₂O, MgCl, MgSO₄, KCl, NaHCO₃, NaCl, Na₂HPO₄, Na₂HPO₄·H₂O, and ZnSO₄·7H₂O.

The organoid culture medium may further contain at least one sugar that can serve as a carbon energy source. Examples of sugars include glucose, galactose, maltose, and fructose. Of these, glucose is preferable, and D-glucose (dextrose) is particularly preferable. The concentration of sugars contained in the organoid culture medium is preferably 1 g/L or more and 10 g/L.

The organoid culture medium may further contain at least one trace element. Examples of trace elements include barium, bromine, cobalt, iodine, manganese, chromium, copper, nickel, selenium, vanadium, titanium, germanium, molybdenum, silicon, iron, fluorine, silver, rubidium, tin, zirconium, cadmium, zinc, aluminum, and ions thereof.

The organoid culture medium may further contain at least one type of additional agent. Examples of such agents include nutrients or growth factors that have been reported to improve stem cell culture, such as cholesterol, transferrin, albumin, insulin, progesterone, putrescine, and selenite.

### [Method for organoid proliferation]

In one embodiment, the present invention provides a method for organoid proliferation, including: a step of culturing an organoid in the presence of a Hippo signaling pathway inhibitor and in the absence of an extracellular matrix. The method for organoid proliferation of the present embodiment can also be said to be a method for producing an organoid.

As will be described below in the examples, the method of the present embodiment enables proliferation of an organoid in the absence of an extracellular matrix. Here, "proliferation" means an increase in the number of cells constituting an organoid and can also be rephrased as "culture."

In the method of the present embodiment, the Hippo signaling pathway inhibitor, the extracellular matrix, the organoid, and the like are the same as those described above. That is, in the method of the present embodiment, the Hippo signaling pathway inhibitor is preferably an MST1/2 kinase inhibitor or a LATS1/2 kinase inhibitor. In addition, in the method of the present embodiment, the organoid is preferably an epithelial organoid.

In the method of the present embodiment, an organoid can also be cultured under serum-free conditions. By enabling proliferation of an organoid in the absence of an extracellular matrix and serum, it is possible to obtain an organoid produced without using any animal-derived products.

### [Organoid]

In one embodiment, the present invention provides an organoid proliferated by the above-described proliferation method.

The organoid of the present embodiment is produced free of animal-derived products. This facilitates its application in regenerative medicine.

The organoid of the present embodiment may be a gastrointestinal organoid. Conventional gastrointestinal organoids cultured in the presence of Matrigel (registered trademark) form a spherical shape in which a monolayer epithelial cell sheet with apical polarity is oriented with the apical side facing inward.

In contrast, as will be described later in the examples, the organoid of the present embodiment has a spherical morphology in which cells are multilayered and the apical polarity of epithelial cells is not maintained, which differs from the morphology of conventional gastrointestinal organoids cultured in the presence of Matrigel (registered trademark).

The organoid of the present embodiment is thought to differ from conventional organoids in terms of gene expression patterns or the like. However, it is unclear whether it is possible to identify differences in gene expression patterns and clearly distinguish between conventional organoids and the organoid of the present embodiment, making it impractical. For this reason, it is considered practical to define the organoid of the present embodiment by the production method.

### [Agent for establishing organoid in absence of extracellular matrix]

In one embodiment, the present invention provides an agent for establishing an organoid in the absence of an extracellular matrix, the agent including a Hippo signaling pathway inhibitor as an active component.

As described above, the inventors have revealed that an organoid can proliferate in the absence of an extracellular matrix by adding a Hippo signaling pathway inhibitor to a culture medium. However, just because an already established organoid could proliferate, it cannot be stated that an organoid can be established from a biologically derived tissue by adding a Hippo signaling pathway inhibitor to a culture medium.

In contrast, as will be described below in the examples, the inventors have revealed that, by adding the agent of the present embodiment to a culture medium, an organoid can be established from a biologically derived tissue in the absence of an extracellular matrix, and that an organoid can also be established from single cells without using an extracellular matrix.

In the agent of the present embodiment, the Hippo signaling pathway inhibitor, the extracellular matrix, the organoid, and the like are the same as those described above. That is, in the method of the present embodiment, the Hippo signaling pathway inhibitor is preferably an MST1/2 kinase inhibitor or a LATS1/2 kinase inhibitor. In addition, in the agent of the present embodiment, the organoid is preferably an epithelial organoid.

### [Method for producing organoid]

In one embodiment, the present invention provides a method for producing an organoid, including: a step of establishing an organoid in the presence of a Hippo signaling pathway inhibitor and in the absence of an extracellular matrix. The method for producing an organoid of the present embodiment can also be said to be a method for establishing an organoid.

As will be described below in the examples, using the method of the present embodiment, an organoid can be established from a biological tissue in the absence of an extracellular matrix, and that an organoid can also be established from single cells without using an extracellular matrix. Furthermore, according to the method of the present embodiment, an organoid can also be established under serum-free conditions. By establishing an organoid in the absence of an extracellular matrix and serum, it is possible to obtain an organoid produced free of animal-derived products from the time of establishment.

In the method of the present embodiment, the Hippo signaling pathway inhibitor, the extracellular matrix, the organoid, and the like are the same as those described above. That is, in the production method of the present embodiment, the Hippo signaling pathway inhibitor is preferably an MST1/2 kinase inhibitor or a LATS1/2 kinase inhibitor. In addition, in the production method of the present embodiment, the organoid is preferably an epithelial organoid.

### [Organoid]

In one embodiment, the present invention provides an organoid produced by the above-described production method.

As will be described below in the examples, the inventors have revealed that the above-described production method makes it possible to establish an organoid from a biologically derived tissue in the absence of an extracellular matrix, and that an organoid can also be established from single cells without using an extracellular matrix. The organoid of the present embodiment, particularly when established under serum-free conditions, is obtained free of animal-derived products from the time of establishment. This makes it particularly easy to transplant into a living body and apply it to regenerative medicine. Therefore, in one embodiment, the present invention provides a regenerative medicine preparation containing, as an active component, an organoid produced by the above-described production method.

The organoid of the present embodiment is thought to differ from conventional organoids in terms of gene expression patterns or the like. However, it is unclear whether it is possible to identify differences in gene expression patterns and clearly distinguish between conventional organoids and the organoid of the present embodiment, making it impractical. For this reason, it is considered practical to define the organoid of the present embodiment by the production method.

### [Method for screening for agent that enables culture of organoid in absence of extracellular matrix]

In one embodiment, the present invention provides a method for screening for an agent that enables culture of an organoid in the absence of an extracellular matrix, the method including: a step of culturing an organoid in the presence of a test substance and in the absence of an extracellular matrix; and a step of evaluating proliferation of the organoid, in which an increase in the proliferation of the organoid compared to when the test substance is absent indicates that the test substance is an agent that enables organoid culture in the absence of an extracellular matrix.

The screening method of the present embodiment makes it possible to screen for an agent that enables organoid culture in the absence of an extracellular matrix.

In the screening method of the present embodiment, the extracellular matrix, the organoid, and the like are the same as those described above.

In the screening method of the present embodiment, the test substance is not particularly limited, and, for example, a natural compound library, a synthetic compound library, an existing drug library, and the like can be used.

The evaluation of the proliferation of an organoid can be carried out through an appropriate method, and examples thereof include evaluation based on measurement of cell number based on microscopic observation, measurement of cell number by measuring ATP, and measurement of cell number using tetrazolium compounds such as MTT. If the proliferation of the organoid is higher compared to when the test substance is absent, the test substance can be determined to be an agent that enables organoid culture in the absence of an extracellular matrix.

### Examples

Examples will be shown below to describe the present invention in more detail, but the present invention is not limited to the following examples.

### [Experimental Example 1]

### (Culture 1 of organoids in presence of LATS kinase inhibitor)

A human small intestine-derived organoid and a human large intestine-derived organoid were cultured in the presence of an inhibitor of LATS1 kinase and LATS2 kinase, Lats-IN-1 (CAS No. 1424635-83-5, hereinafter sometimes referred to as "Lats-IN" and also referred to as "TRULI"), and their dynamics were observed.

The human small intestine-derived organoid and the human large intestine-derived organoid used were those established from tissues derived from patients with gastrointestinal tumors who provided informed consent, in accordance with an ethical research plan approved by the Ethics Committee of the Keio University School of Medicine.

Each organoid was dissociated into single cells using TrypLE Express (Thermo Fisher Scientific Inc.) to obtain a cell suspension. Subsequently, 1×10⁵ cells/well were seeded in a 48-well plate with 25 µL of Matrigel (registered trademark). Subsequently, after the Matrigel (registered trademark) had gelled, 100 µL/well of a medium having the composition shown in Table 1 below was added thereto, and the cells were cultured at 37°C. 10 mM Lats-IN was added to the medium for the test group. Subsequently, the medium was replaced every 2 days.

**[Table 1]**

| Composition of medium |
|---|
| Advanced DMEM/F-12 medium |
| 10 mM HEPES |
| 2 mM GlutaMAX |
| 100U/mL Penicillin / 100 µg/mL streptomycin (Thermo Fisher Scientific Inc.) |
| 1 mM N-acetylcysteine (FUJIFILM Wako Pure Chemical Corporation) |
| 1×B27 (Thermo Fisher Scientific Inc.) |
| 10 nM Gastrin I (Sigma-Aldrich Co. LLC.) |
| 5% afamin-Wnt-3A serum-free conditioned medium |
| 50 ng/mL Mouse recombinant EGF (Thermo Fisher Scientific Inc.) |
| 100 ng/mL Mouse recombinant Noggin (PeproTech, Inc.) |
| 10% R-Spondin 1 conditioned medium |
| 10 nM A83-01 (R&D Systems) |
| 10 µM Y-27632 (FUJIFILM Wako Pure Chemical Corporation) |
| 50 µg/mL Human FGF-2 (PeproTech, Inc.) |
| 50 ng/mL Human FGF-10 (PeproTech, Inc.) |
| 100 µg/mL Human IGF-1 (BioLegend, Inc.) |

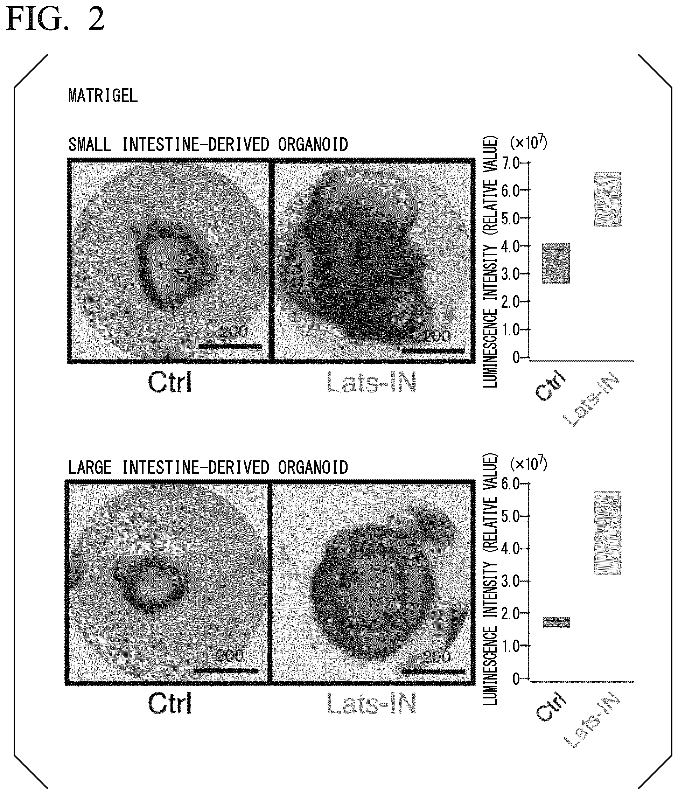

FIG. 2 shows representative photographs and graphs for the organoids, with the graphs showing their cell number after 7 days of culture. The upper row of FIG. 2 shows the results for human small intestine-derived organoids, and the lower row of FIG. 2 shows the results for human large intestine-derived organoids. The cell proliferation was measured by measuring adenosine triphosphate (ATP) using a commercially available kit (product name "CellTiter-Glo 3D," Promega Corporation).

The upper left panel of FIG. 2 is a photograph of a control (Ctrl) human small intestine-derived organoid cultured in the absence of Lats-IN, and the upper center panel of FIG. 2 is a photograph of a human small intestine-derived organoid cultured in the presence of Lats-IN. The scale bar is 200 µm. The upper right panel of FIG. 2 is a graph showing the results of measuring cell number per well in the human small intestine-derived organoids cultured in the presence of the control (Ctrl) and Lats-IN. The vertical axis of the graph represents luminescence intensity (relative value) indicating the amount of ATP present.

The lower left panel of FIG. 2 is a photograph of a control (Ctrl) human large intestine-derived organoid cultured in the absence of Lats-IN, and the lower center panel of FIG. 2 is a photograph of a human large intestine-derived organoid cultured in the presence of Lats-IN. The scale bar is 200 µm. The lower right panel of FIG. 2 is a graph showing the results of measuring cell number per well in the human large intestine-derived organoids cultured in the presence of the control (Ctrl) and Lats-IN. The vertical axis of the graph represents luminescence intensity (relative value) indicating the amount of ATP present.

As a result, it was revealed that cell proliferation was significantly activated in both the human small intestine-derived and human large intestine-derived organoids by adding the LATS kinase inhibitor to the media.

### [Experimental Example 2]

### (Culture 2 of organoids in presence of LATS kinase inhibitor)

Conventionally, extracellular matrices such as Matrigel (registered trademark) are essential for organoid culture, and it has been difficult to sufficiently maintain cell proliferation and differentiation using alternative substances to Matrigel (registered trademark).

In addition, when cells are cultured three-dimensionally in the complete absence of an extracellular matrix such as Matrigel (registered trademark), they aggregate to form cell masses, but in this state the cell proliferation activity is very low, making continuous passaging completely impossible.

In this experimental example, a human small intestine-derived organoid and human large intestine-derived organoid established in the presence of Matrigel (registered trademark) were cultured in the absence of Matrigel (registered trademark) and in the presence of a LATS kinase inhibitor (final concentration: 10 µM), and their dynamics were observed. The procedure was the same as in Experimental Example 1, except that each organoid was cultured in the absence of Matrigel (registered trademark).

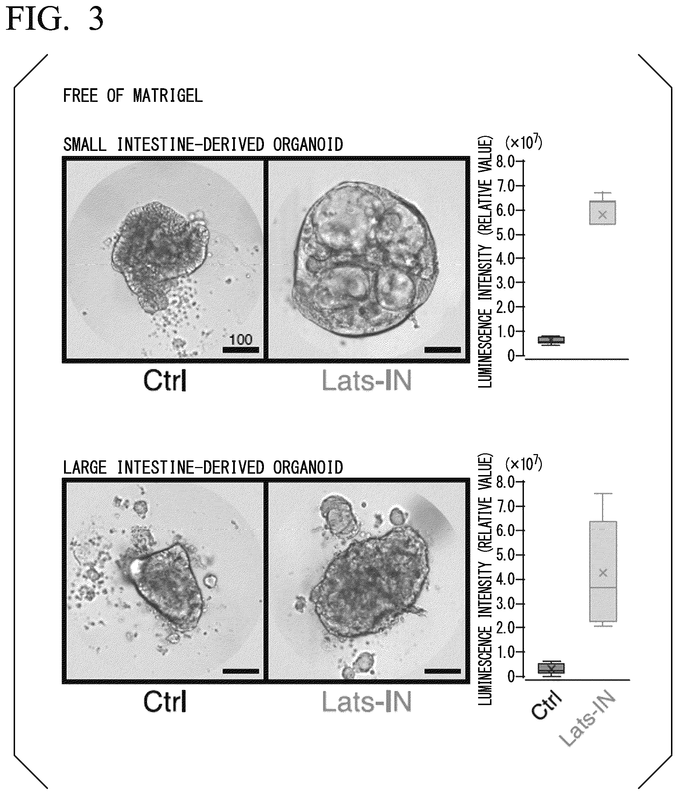

FIG. 3 shows representative photographs and graphs for organoids, with the graphs showing their cell number after 7 days of culture. The upper row of FIG. 3 shows the results for human small intestine-derived organoids, and the lower row of FIG. 3 shows the results for human large intestine-derived organoids.

The upper left panel of FIG. 3 is a photograph of a control (Ctrl) human small intestine-derived organoid cultured in the absence of Lats-IN, and the upper center panel of FIG. 3 is a photograph of a human small intestine-derived organoid cultured in the presence of Lats-IN. The scale bar is 100 µm. The upper right panel of FIG. 3 is a graph showing the results of measuring cell number in the human small intestine-derived organoids cultured in the presence of the control (Ctrl) and Lats-IN by measuring ATP using a commercially available kit (product name "CellTiter-Glo 3D," Promega Corporation). The vertical axis of the graph represents luminescence intensity (relative value) indicating the amount of ATP present.

The lower left panel of FIG. 3 is a photograph of a control (Ctrl) human large intestine-derived organoid cultured in the absence of Lats-IN, and the lower center panel of FIG. 3 is a photograph of a human large intestine-derived organoid cultured in the presence of Lats-IN. The scale bar is 100 µm. The lower right panel of FIG. 3 is a graph showing the results of measuring cell number in the human large intestine-derived organoids cultured in the presence of the control (Ctrl) and Lats-IN. The vertical axis of the graph represents luminescence intensity (relative value) indicating the amount of ATP present.

As a result, it was revealed that cell proliferation was significantly activated in both the human small intestine-derived and human large intestine-derived organoids by the addition of the LATS kinase inhibitor to the media even in the absence of Matrigel (registered trademark). The fact that organoid proliferation became possible even in the absence of Matrigel (registered trademark), which was previously considered impossible, is a remarkable finding.

### [Experimental Example 3]

### (Examination of phosphorylation of YAP)

Human small intestine-derived organoids were cultured in the absence and presence of Matrigel (registered trademark) and in the absence and presence of a LATS kinase inhibitor (final concentration: 10 µM), and phosphorylation of YAP was confirmed by Western blotting.

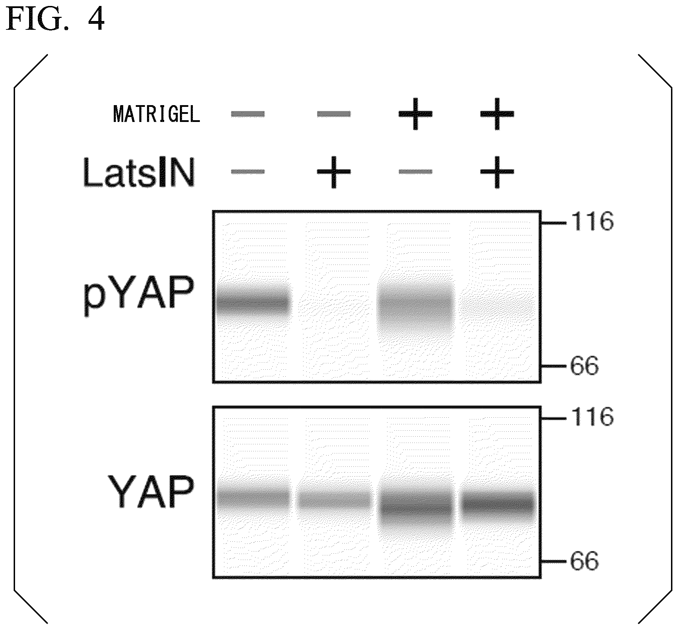

FIG. 4 shows representative photographs showing the results of Western blotting. **In** FIG. 4, "pYAP" indicates phosphorylated YAP, and "YAP" indicates total YAP (the sum of phosphorylated YAP and non-phosphorylated YAP). The antibody against phosphorylated YAP used was phospho-YAP (Ser127) (#4911, Cell Signaling Technology, Inc.). The antibody against total YAP used was total-YAP (#14074, Cell Signaling Technology, Inc.).

As a result, it was confirmed that phosphorylation of YAP was significantly suppressed in the presence of Lats-IN compared to when Lats-IN is absent, in both the presence and absence of Matrigel (registered trademark).

### [Experimental Example 4]

### (Examination by immunostaining)

Human small intestine-derived organoids and human large intestine-derived organoids were cultured in the absence and presence of Matrigel (registered trademark) and in the absence and presence of a LATS kinase inhibitor (final concentration: 10 µM) in the same manner as in Experimental Example 2. Subsequently, immunostaining was performed to confirm the localization of YAP. In addition, Ki67, a marker for cell proliferation, was detected to confirm proliferating cells.

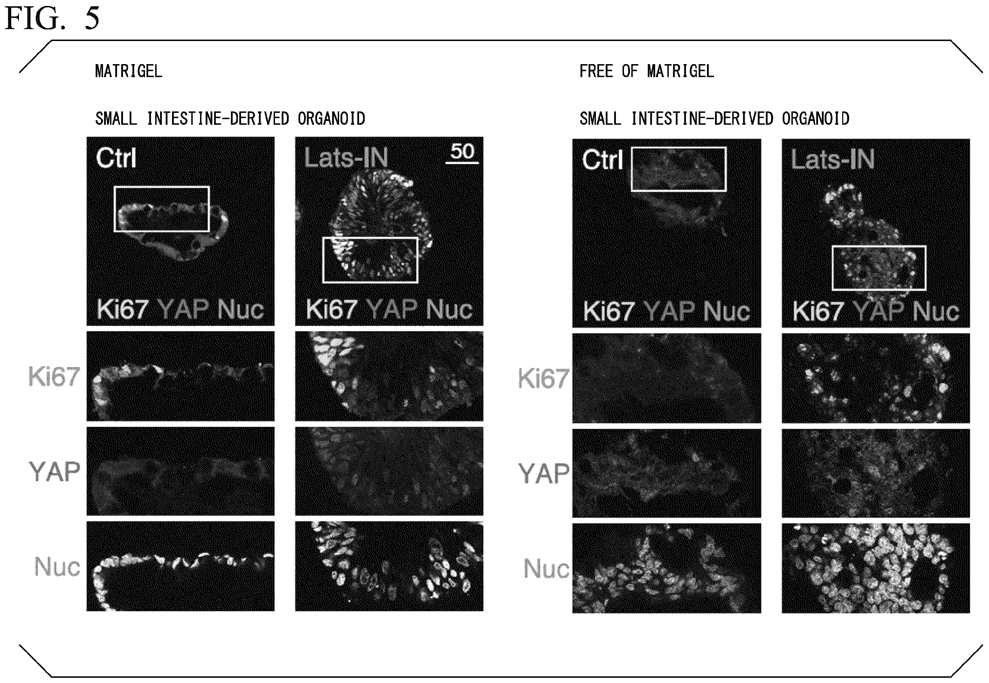

The leftmost column of FIG. 5 shows photographs showing the results of immunostaining of the human small intestine-derived organoids cultured in the presence of Matrigel (registered trademark) and in the absence of Lats-IN (Ctrl). The uppermost panel shows images in which the detection results of Ki67, YAP, and the nucleus (Nuc) are merged, the second panel from the top shows images showing the detection results of Ki67, the third panel from the top shows images showing the detection results of YAP, and the fourth panel from the top shows images showing the detection results of the nucleus (Nuc). The fields of view in the second, third, and fourth panels from the top correspond to the area enclosed in the square in the uppermost panel.

The second column from the left of FIG. 5 shows photographs showing the results of immunostaining of the human small intestine-derived organoids cultured in the presence of Matrigel (registered trademark) and Lats-IN. The uppermost panel shows images in which the detection results of Ki67, YAP, and the nucleus (Nuc) are merged, the second panel from the top shows images showing the detection results of Ki67, the third panel from the top shows images showing the detection results of YAP, and the fourth panel from the top shows images showing the detection results of the nucleus (Nuc). The fields of view in the second, third, and fourth panels from the top correspond to the area enclosed in the square in the uppermost panel. The scale bar represents 50 µm.

The third column from the left of FIG. 5 presents photographs showing the results of immunostaining of the human small intestine-derived organoids cultured in the absence of Matrigel (registered trademark) and Lats-IN (Ctrl). The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

The rightmost column of FIG. 5 presents photographs showing the results of immunostaining of the human small intestine-derived organoids cultured in the absence of Matrigel (registered trademark) and in the presence of Lats-IN. The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

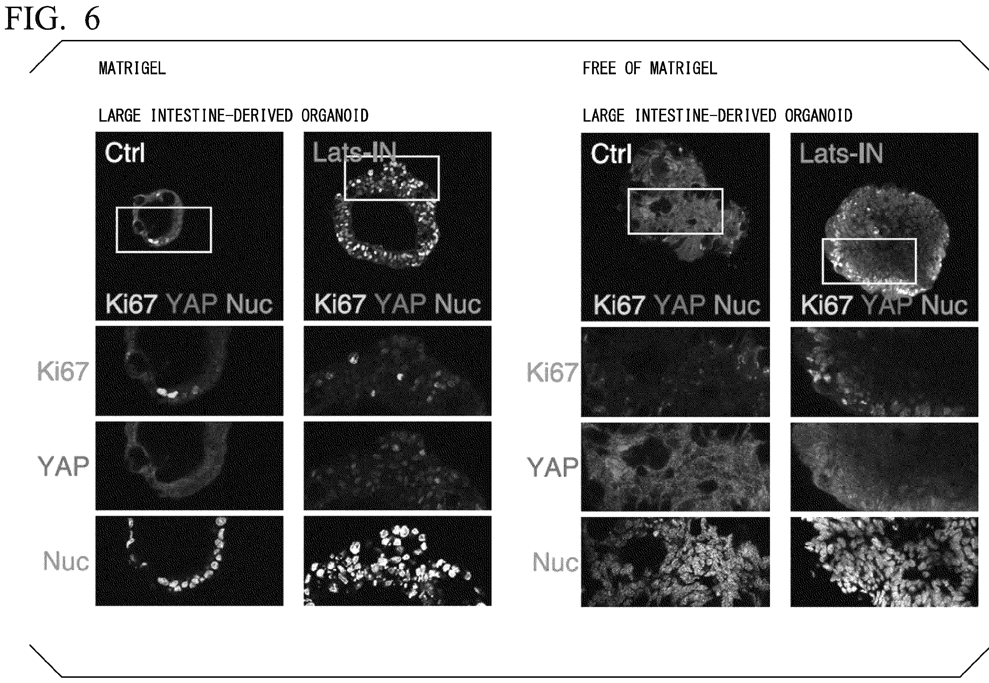

The leftmost column of FIG. 6 presents photographs showing the results of immunostaining of the human large intestine-derived organoids cultured in the presence of Matrigel (registered trademark) and in the absence of Lats-IN (Ctrl). The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

The second column from the left of FIG. 6 presents photographs showing the results of immunostaining of the human large intestine-derived organoids cultured in the presence of Matrigel (registered trademark) and Lats-IN. The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

The third column from the left of FIG. 6 presents photographs showing the results of immunostaining of the human large intestine-derived organoids cultured in the absence of Matrigel (registered trademark) and Lats-IN (Ctrl). The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

The rightmost column of FIG. 6 presents photographs showing the results of immunostaining of the human large intestine-derived organoids cultured in the absence of Matrigel (registered trademark) and in the presence of Lats-IN. The uppermost panel shows images in which the detection results of Ki67, YAP, and the nucleus (Nuc) are merged, the second panel from the top presents images showing the detection results of Ki67, the third panel from the top presents images showing the detection results of YAP, and the fourth panel from the top presents images showing the detection results of the nucleus (Nuc). The fields of view in the second, third, and fourth panels from the top correspond to the area enclosed in the square in the uppermost panel.

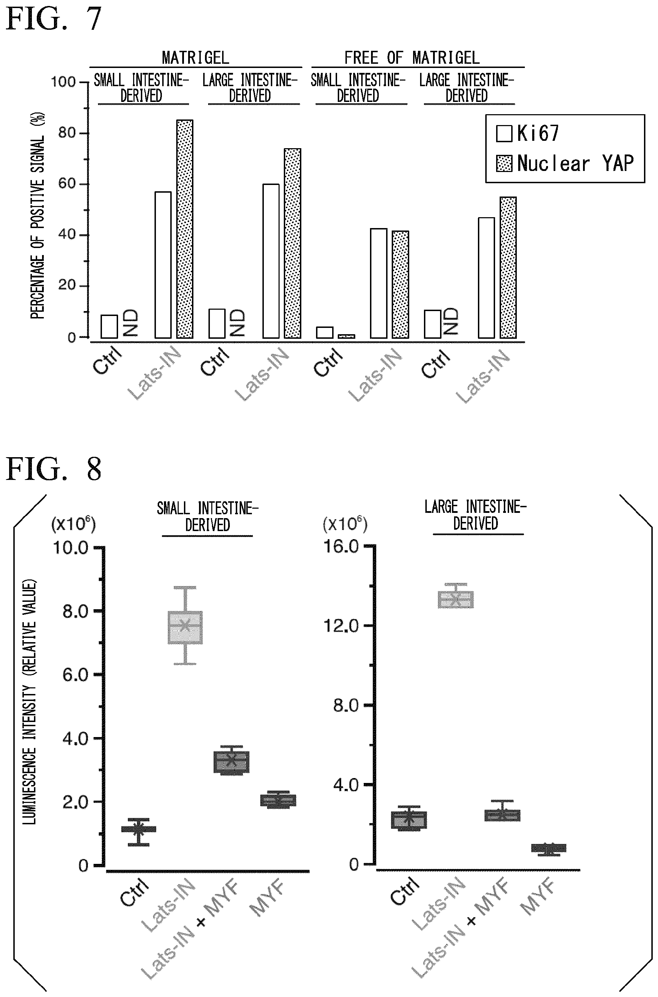

FIG. 7 is a graph summarizing the results of FIGS. 5 and 6. As a result, in both the presence and absence of Matrigel (registered trademark), in the absence of Lats-IN, YAP was localized in the cytoplasm, and when the number of Ki67-positive cells, which indicates cell proliferation, was counted, the percentage of Ki67-positive cells was approximately 10% of the total cells. In contrast, in both the presence and absence of Matrigel (registered trademark), in the presence of Lats-IN, the number of cells in which YAP was localized in the nucleus was counted. As a result, the proportion of cells in which YAP was localized in the nucleus was approximately 50% to 80% of the total cells, and the percentage of Ki67-positive cells was approximately 50% or more of the total cells.

The above results indicate that the addition of Lats-IN inhibits LATS, inhibits phosphorylation of YAP, translocates YAP into the nucleus, and activates cell proliferation.

### [Experimental Example 5]

### (Examination of transcriptional activity of TEAD)

It was examined whether YAP that has translocated into the nucleus by the addition of Lats-IN supports the transcriptional activity of TEAD. Specifically, human small intestine-derived organoids and human large intestine-derived organoids were cultured in the absence of Matrigel (registered trademark) and in the absence or presence of Lats-IN (final concentration: 10 µM), and cell proliferation was observed when TEAD was inhibited using MYF01-37 (CAS No. 2416417-65-5), a TEAD inhibitor.

FIG. 8 presents graphs showing the results of measuring the proliferation of the human small intestine-derived and human large intestine-derived organoids in the absence of Lats-IN (Ctrl), in the presence of Lats-IN (Lats-IN), in the presence of Lats-IN and MYF01-37 (Lats-IN + MYF), and in the presence of MYF01-37 (MYF). The cell proliferation was measured by measuring ATP using a commercially available kit (product name "CellTiter-Glo 3D," Promega Corporation). The vertical axis of the graph represents luminescence intensity (relative value) indicating the amount of ATP present.

In both types of organoids, the results showed that cell proliferation was activated in the group to which Lats-IN was added, whereas cell proliferation was inhibited in the group to which both Lats-IN and MYF01-37 were added.

These results indicate that the addition of Lats-IN inhibits the function of LATS, causing YAP to translocate into the nucleus, and that the YAP that has translocated into the nucleus supports the transcriptional activity of TEAD. In other words, it was shown that the addition of Lats-IN to the media enables cell proliferation in the absence of Matrigel (registered trademark), which was previously impossible, through pathways of LATS inhibition, YAP nuclear translocation, and TEAD-mediated promotion of the transcriptional activity of cell growth factors.

### [Experimental Example 6]

### (Examination of organoid formation from single cells)

It was examined whether organoids could be formed from single cells in the absence of Matrigel (registered trademark). Cell-substrate interaction has been thought to be essential for cell survival and proliferation, but cell-substrate interactions do not exist when cultured in the absence of Matrigel (registered trademark). This revealed that signals from cell-substrate interactions are not involved in the transcriptional regulation of YAP-TEAD by LATS inhibition.

In addition to cell-substrate interactions, signals from cell-to-cell interactions are thought to play an important role in cell survival and proliferation.

Therefore, to examine whether signals from cell-to-cell interactions are involved in cell proliferation mediated by transcriptional regulation of YAP-TEAD by LATS inhibition, human small intestine-derived organoids were dissociated into single cells and then observed organoid formation from the single cells.

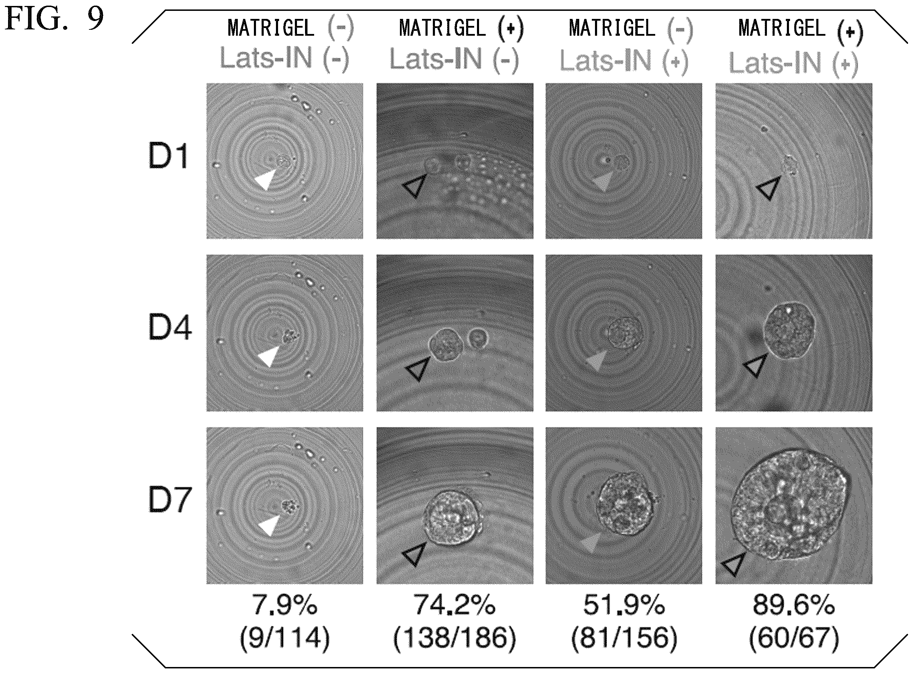

FIG. 9 presents representative photographs showing the results of organoid formation from single cells in the presence or absence of Matrigel (registered trademark) and in the absence or presence of Lats-IN (final concentration: 10 µM). In FIG. 9, the arrowheads indicate cells or organoids. In addition, D1, D4, and D7 indicate the first, fourth, and seventh days, respectively, after the start of culture. In the parentheses in the lower row of FIG. 9, the denominator shows the number of single cells seeded, and the numerator shows the number of organoids formed. **In** addition, the percentage (%) of cells that formed organoids among the seeded single cells is also shown.

The results showed that cells hardly proliferated in the absence of Matrigel (registered trademark) and Lats-IN. In contrast, it was revealed that cells proliferated from single cells and formed organoids in the presence of Matrigel (registered trademark), and in the absence of Matrigel (registered trademark) and in the presence of Lats-IN. In addition, it was also shown that the percentage of cells that formed organoids was higher in the presence of Matrigel (registered trademark) and Lats-IN than in the absence of Matrigel (registered trademark) and in the presence of Lats-IN.

The above results suggested that transcriptional regulation of YAP-TEAD by LATS inhibition does not involve signals from cell-to-cell interactions, and that the transcriptional regulation of YAP-TEAD by LATS inhibition activates cell proliferation and contributes to organoid formation through the signal alone.

### [Experimental Example 7]

### (Organoid establishment in absence of Matrigel (registered trademark))

It was examined whether organoids could be established from the human lower gastrointestinal tract in the absence of Matrigel (registered trademark).

Crypts were isolated from human small intestine- and large intestine-derived tissues. The isolated crypts were cultured in the absence of Matrigel (registered trademark) and in the absence or presence of Lats-IN (final concentration: 10 µM).

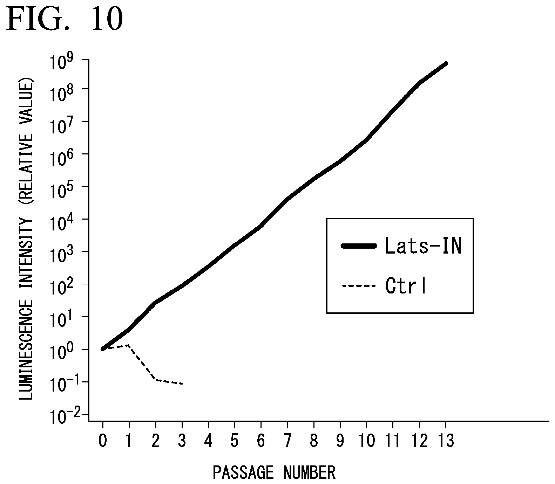

FIG. 10 is a graph showing the results of a time-course measurement of proliferation of the crypts isolated from the human large intestine-derived tissue over time. The cell proliferation was measured by measuring ATP using a commercially available kit (product name "CellTiter-Glo 3D," Promega Corporation). The vertical axis of the graph represents luminescence intensity (relative value) indicating the amount of ATP present. As shown in FIG. 10, in the absence of Lats-IN, the isolated crypts increased in number enough to undergo one passage, but then gradually underwent apoptosis and completely died after the second passage.

In contrast, in the presence of Lats-IN, the isolated crypts were able to undergo passage almost continuously without cessation of cell proliferation. Similar results were obtained also in crypts isolated from human small intestine-derived tissue.

The above results showed that in the presence of Lats-IN, LATS is inhibited, leading to YAP-induced cell proliferation, and enabling organoid establishment even in the absence of Matrigel (registered trademark).

### [Experimental Example 8]

### (Culture of organoids derived from various organs)

It was examined whether organoids derived from various organs could be cultured in the absence of Matrigel (registered trademark) and in the absence or presence of Lats-IN (final concentration: 10 µM).

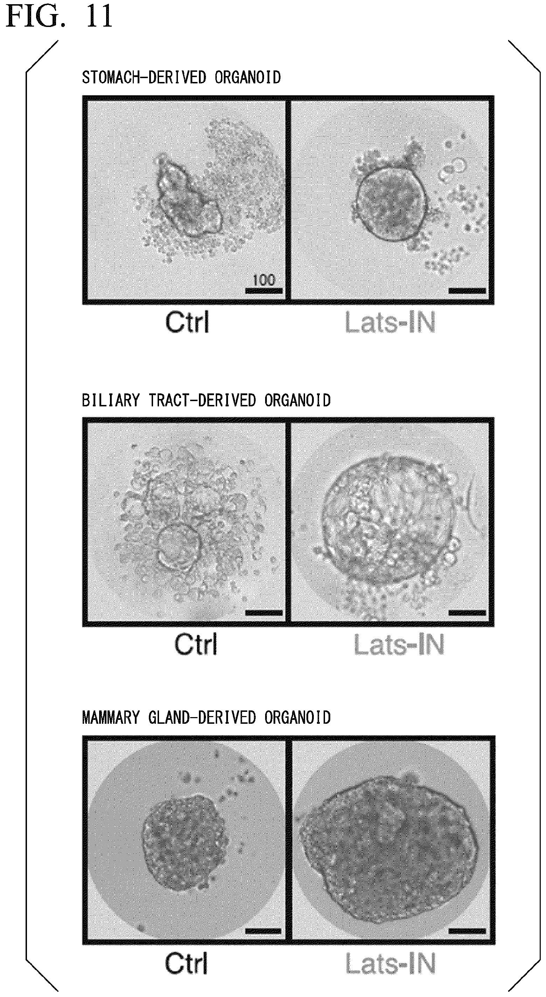

FIG. 11 presents representative photographs showing the results of culturing stomach-derived organoids, biliary tract-derived organoids, and mammary gland-derived organoids in the absence of Matrigel (registered trademark) and Lats-IN (Ctrl), or in the absence of Matrigel (registered trademark) and in the presence of Lats-IN (Lats-IN). The scale bar is 100 µm.

The results showed that cell proliferation was activated in the presence of Lats-IN even in the absence of Matrigel (registered trademark), and that organoids derived from all organ, not just the human lower gastrointestinal tract, can be cultured.

### [Experimental Example 9]

### (Examination 1 of organoid morphology)

Human lower gastrointestinal organoids established in the presence of Matrigel (registered trademark) were cultured in the absence of Matrigel (registered trademark) and in the presence of Lats-IN (final concentration 10: µM), and their morphology and properties were observed.

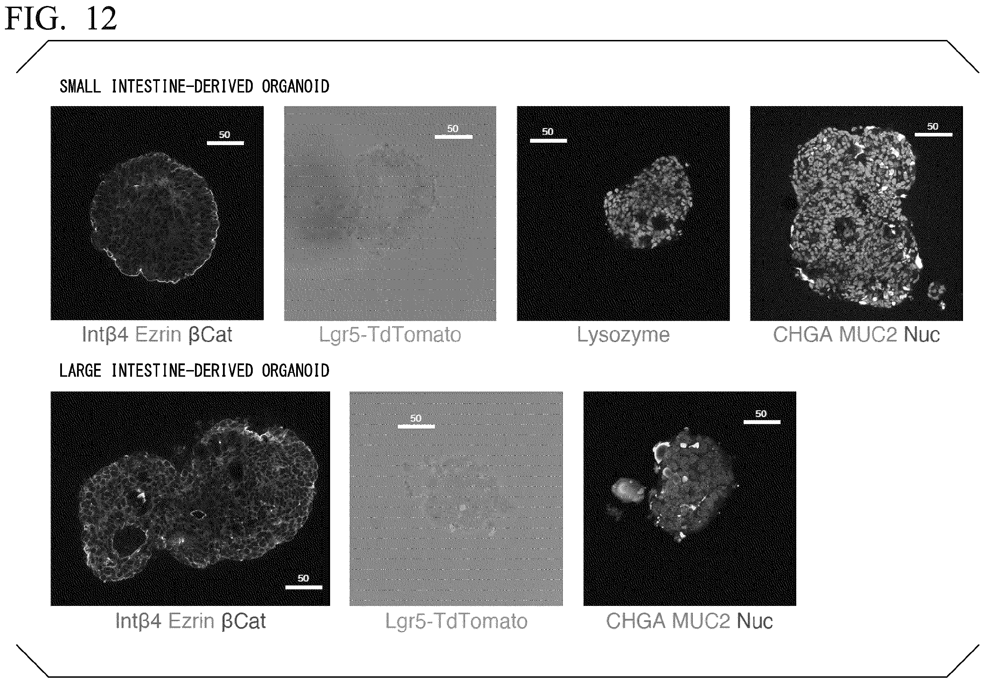

The upper row of FIG. 12 shows representative photographs of immunostained human small intestine-derived organoids. The lower row of FIG. 12 shows representative photographs of immunostained human large intestine-derived organoids. In FIG. 12, the scale bar represents 50 µm.

Immunostaining detected the basement membrane marker integrin β4 (Intβ4), the apical marker ezrin, the cell-cell adhesion marker β-catenin (βCat), the Paneth cell marker lysozyme, the embryonic cell marker mucin 2 (MUC2), the enteroendocrine cell marker chromogranin A (CHGA), and the nucleus (Nuc). In addition, expression of the stem cell marker Lgr5 was detected by the fluorescence of TdTomato, a fluorescent protein introduced into the Lgr5 gene locus of the cells.

Conventional human lower gastrointestinal organoids cultured in the presence of Matrigel (registered trademark) form a spherical shape in which a monolayer epithelial cell sheet with apical polarity is oriented with the apical side facing inward, and each organoid contains stem cells and differentiated cells such as embryonic cells and enteroendocrine cells.

In contrast, as shown in FIG. 12, the human lower gastrointestinal organoids cultured in the absence of Matrigel (registered trademark) and in the presence of Lats-IN had a spherical morphology in which cells are multilayered and the apical polarity of the epithelial cells was not maintained, which was significantly different from the morphology of the conventional human lower gastrointestinal organoids cultured in the presence of Matrigel (registered trademark).

However, as shown in FIG. 12, the human lower gastrointestinal organoids cultured in the absence of Matrigel (registered trademark) and in the presence of Lats-IN were confirmed to express not only the stem cell marker Lgr5, but also the Paneth cell marker lysozyme, the embryonic cell marker Mucin 2, and the enteroendocrine cell marker chromogranin A. This indicated that, like the conventional human lower gastrointestinal organoids cultured in the presence of Matrigel (registered trademark), each organoid contains stem cells and differentiated cells.

### [Experimental Example 10]

### (Examination 2 of organoid morphology)

Human lower gastrointestinal organoids established in the presence of Matrigel (registered trademark) were cultured for seven passages in the absence of Matrigel (registered trademark) and in the presence of Lats-IN (final concentration 10: µM). Subsequently, they were cultured again in the presence of Matrigel (registered trademark), and their morphology and properties were observed.

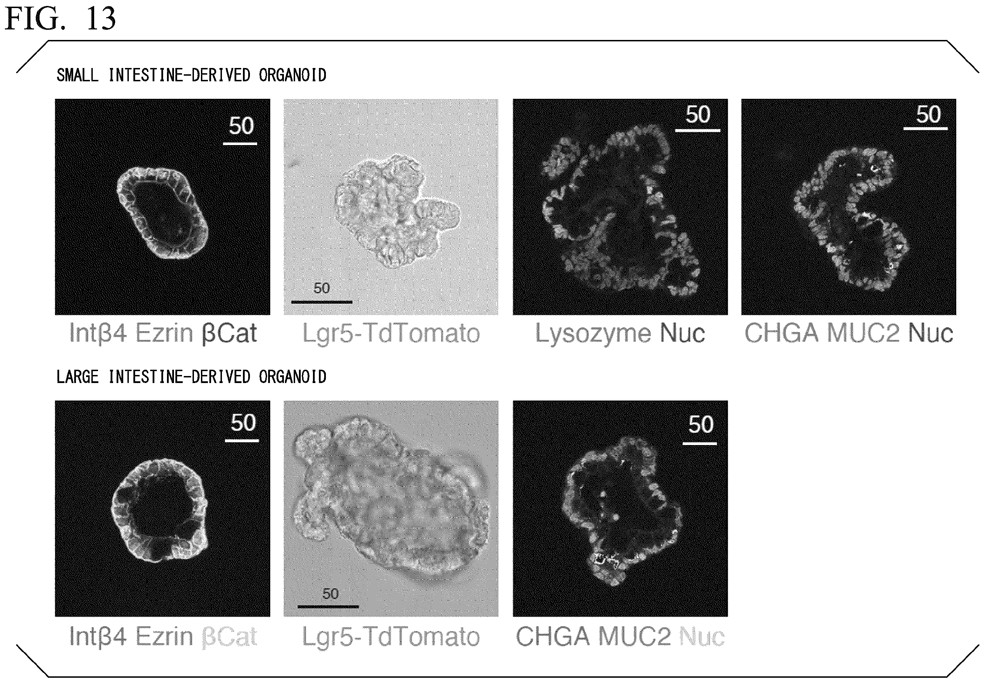

The upper row of FIG. 13 shows representative photographs of immunostained human small intestine-derived organoids. The lower row of FIG. 13 shows representative photographs of immunostained human large intestine-derived organoids. In FIG. 13, the scale bar represents 50 µm.

Immunostaining detected the basement membrane marker integrin β4 (Intβ4), the apical marker ezrin, the cell-cell adhesion marker β-catenin (βCat), the Paneth cell marker lysozyme, the embryonic cell marker mucin 2 (MUC2), the enteroendocrine cell marker chromogranin A (CHGA), and the nucleus (Nuc). In addition, expression of the stem cell marker Lgr5 was detected by the fluorescence of TdTomato, a fluorescent protein introduced into the Lgr5 gene locus of the cells.

As a result, as shown in FIG. 13, even the human lower gastrointestinal organoids cultured in the absence of Matrigel (registered trademark) and in the presence of Lats-IN, when subsequently cultured again in the presence of Matrigel (registered trademark), formed a spherical shape in which a monolayer epithelial cell sheet with apical polarity is oriented with the apical side facing inward, and it was revealed that each organoid contained stem cells and differentiated cells such as embryonic cells and enteroendocrine cells.

This result indicated that the properties of the human lower gastrointestinal epithelial cells were maintained even after long-term culture in the absence of Matrigel (registered trademark).

### [Experimental Example 11]

### (Investigation of niche factor essential for organoid culture)

The inventors have previously revealed that niche factors essential for culture of the human lower gastrointestinal epithelial organoids are a Wnt agonist (W), R-spondin (R), EGF (E), a BMP inhibitor (N) such as noggin, a TGF-β inhibitor (A) such as A83-01, IGF1 (I), and FGF2 (F).

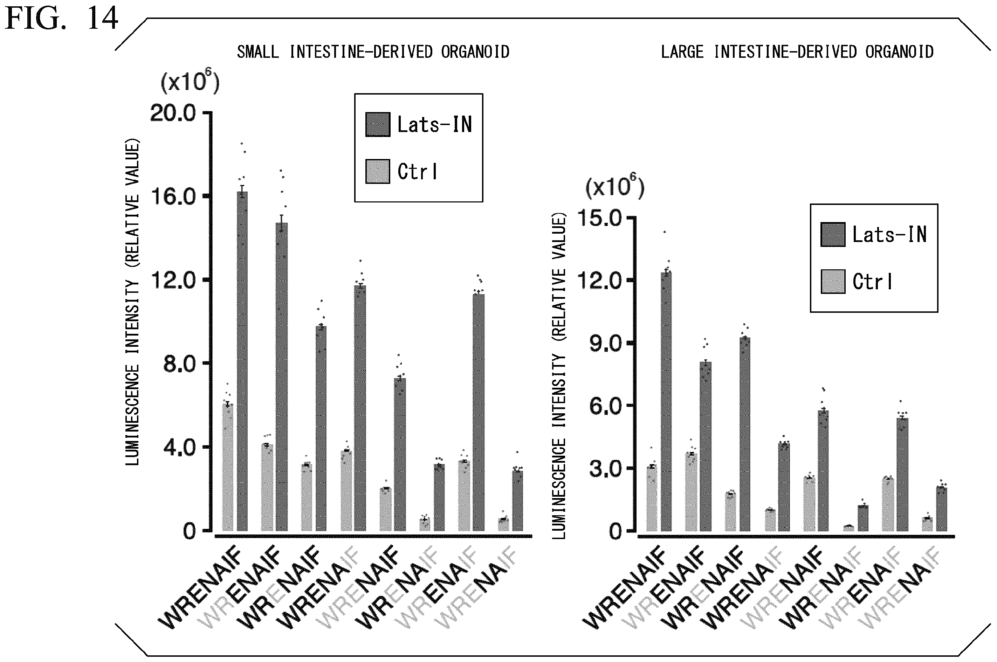

In this experimental example, the relationship between LATS inhibition and the niche factors essential for organoid culture was examined. FIG. 14 presents graphs showing the results of measuring the proliferation of human small intestine-derived organoids and human large intestine-derived organoids cultured under conditions in which all WRENAIF niche factors were added, and under conditions in which some of these niche factors were removed. As a medium to which all of the WRENAIF niche factors were added, the medium whose composition is shown in Table 1 above was used, and as a medium from which some of these niche factors were removed, a medium from which the corresponding niche factors were removed from the medium whose composition is shown in Table 1 above was used. The cell proliferation was measured by measuring ATP using a commercially available kit (product name "CellTiter-Glo 3D," Promega Corporation). The vertical axis of the graph represents luminescence intensity (relative value) indicating the amount of ATP present.

In FIG. 14, "Ctrl" shows the results of culturing in the presence of Matrigel (registered trademark), and "Lats-IN" shows the results of culturing in the absence of Matrigel (registered trademark) and in the presence of Lats-IN (final concentration: 10 µM). In addition, on the horizontal axis, added niche factors are shown in black, and niche factors not added are shown in light color.

The results showed that cells proliferated by adding Lats-IN, even without adding one or two niche factors required for human lower gastrointestinal epithelial organoid culture to the culture media.

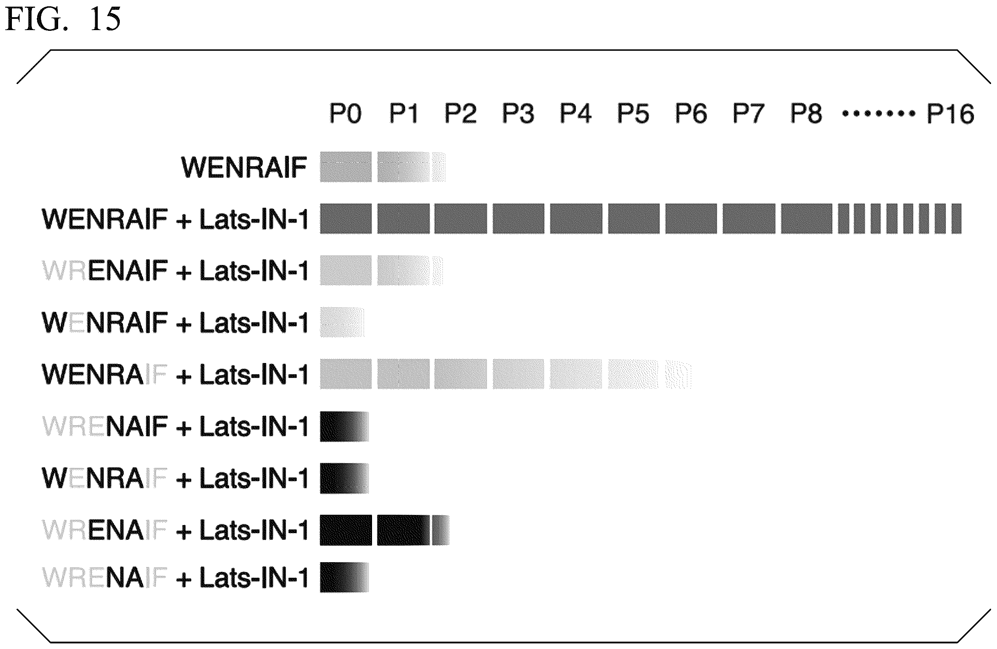

FIG. 15 is a graph showing the results of measuring the proliferation of human small intestine-derived organoids cultured in the absence of Matrigel (registered trademark), in the absence or presence of Lats-IN (final concentration: 10 µM), under conditions in which all WRENAIF niche factors were added, and under conditions in which some of these niche factors were removed. In FIG. 15, P0 to P16 indicate passage numbers 0 to 16. In addition, the dark colors in the graph indicate successful passaging, while the light colors indicate unsuccessful passaging.

As a result, even if Lats-IN was added, continuous passaging was not possible when cultured in a culture medium lacking any of the WRENAIF niche factors. The above results revealed that Lat-IN does not complement the niche factors essential for organoid culture, but rather cooperates with the niche factors to promote the organoid proliferation.

### [Experimental Example 12]

### (Examination of mechanism by which organoid culture is possible in absence of Matrigel (registered trademark))

It was examined why inhibition of LATS enables organoid culture in the absence of Matrigel (registered trademark).

Human small intestine-derived organoids and human large intestine-derived organoids were cultured in the absence or presence of Matrigel (registered trademark) and in the absence or presence of Lats-IN (final concentration: 10 µM), and the expression level of the AXIN2 gene, whose expression is regulated by Wnt, was examined by quantitative real-time PCR.

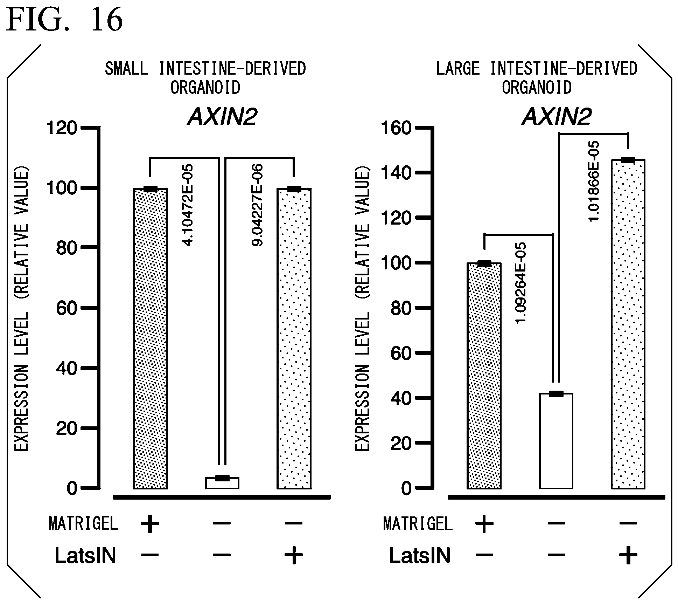

FIG. 16 presents graphs showing the results of quantitative real-time PCR. The results showed that expression of AXIN2 was observed when organoids were cultured in the presence of Matrigel (registered trademark), whereas the expression level of AXIN2 was significantly reduced when organoids were cultured in the absence of Matrigel (registered trademark). Furthermore, it was shown that when organoids were cultured in the absence of Matrigel (registered trademark) and in the presence of Lats-IN, the expression level of AXIN2 increased to a level equivalent to that in the presence of Matrigel (registered trademark).

Next, human small intestine-derived organoids were cultured in the absence or presence of Matrigel (registered trademark) and in the absence or presence of Lats-IN (final concentration: 10 µM), and ERK phosphorylated by EGF signaling was detected by Western blotting.

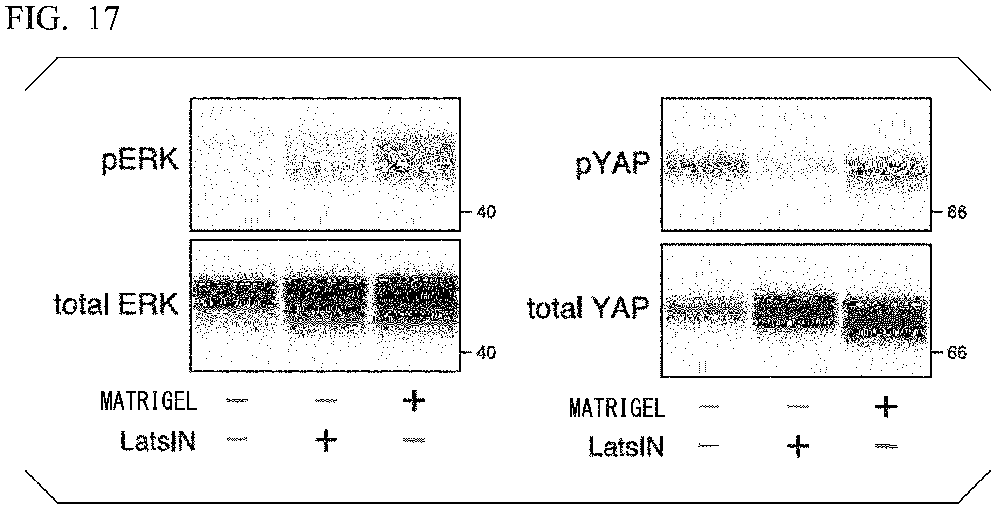

FIG. 17 presents graphs showing the results of Western blotting. In FIG. 17, "pERK" represents phosphorylated ERK, and "total ERK" represents total ERK (the sum of phosphorylated ERK and non-phosphorylated ERK). In addition, "pYAP" represents phosphorylated YAP, and "total YAP" represents total YAP (the sum of phosphorylated YAP and non-phosphorylated YAP). The antibody against phosphorylated ERK used was phospho-ERK (Thr202/Tyr204) (#4370, Cell Signaling Technology, Inc.). The antibody against total ERK used was total-ERK (#4695, Cell Signaling Technology, Inc.). The antibody against phosphorylated YAP used was phospho-YAP (Ser127) (#4911, Cell Signaling Technology, Inc.). The antibody against total YAP used was total-YAP (#14074, Cell Signaling Technology, Inc.).

The results showed that ERK phosphorylation was observed when organoids were cultured in the presence of Matrigel (registered trademark), whereas ERK phosphorylation was significantly reduced when organoids were cultured in the absence of Matrigel (registered trademark). Furthermore, it was shown that when organoids were cultured in the absence of Matrigel (registered trademark) and in the presence of Lats-IN, the ERK phosphorylation increased to a level equivalent to that in the presence of Matrigel (registered trademark).

The above results showed that Wnt and EGF signals, which do not enter cells in the absence of Matrigel (registered trademark), can enter cells even in the absence of Matrigel (registered trademark) by inhibiting LATS, thereby enabling cell proliferation and thus organoid culture.

### [Experimental Example 13]

### (Examination 3 of organoid morphology)

Human large intestine-derived organoids were established in the absence of Matrigel (registered trademark) and in the presence of Lats-IN (final concentration 10: µM) in the same manner as in Experimental Example 7, and their morphology and properties were observed. This experimental example was different from Experimental Example 9 in that the organoids were free of Matrigel (registered trademark) from the time of their establishment.

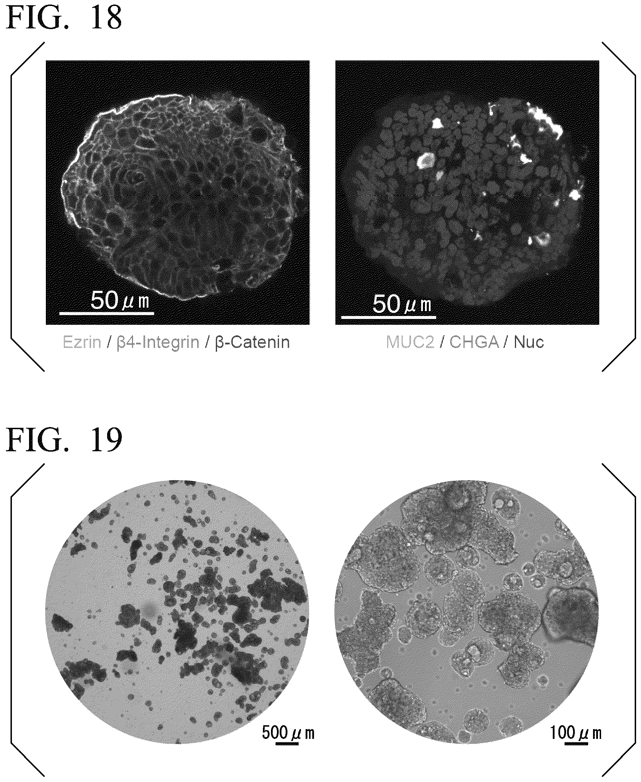

FIG. 18 shows representative photographs of immunostained human large intestine-derived organoids on day 7 of culture, which had undergone 18 passages since establishment. In FIG. 18, the scale bar represents 50 µm.

Immunostaining detected the apical marker ezrin, the basement membrane marker integrin β4 (β4-Integrin), the cell-cell adhesion marker β-catenin (β-Catenin), the embryonic cell marker mucin 2 (MUC2), the enteroendocrine cell marker chromogranin A (CHGA), and the nucleus (Nuc).

As described above, conventional human lower gastrointestinal organoids cultured in the presence of Matrigel (registered trademark) form a spherical shape in which a monolayer epithelial cell sheet with apical polarity is oriented with the apical side facing inward, and each organoid contains stem cells and differentiated cells such as embryonic cells and enteroendocrine cells.

In contrast, as shown in FIG. 18, the human large intestine-derived organoids established in the absence of Matrigel (registered trademark) and in the presence of Lats-IN had a spherical morphology in which cells are multilayered and the apical polarity of the epithelial cells was not maintained, which was significantly different from the morphology of the conventional human lower gastrointestinal organoids cultured in the presence of Matrigel (registered trademark).

However, as shown in FIG. 18, the human lower gastrointestinal organoids established in the absence of Matrigel (registered trademark) and in the presence of Lats-IN were confirmed to express the embryonic cell marker Mucin 2 and the enteroendocrine cell marker chromogranin A. This was thought that, like the conventional human lower gastrointestinal organoids established in the presence of Matrigel (registered trademark), each organoid contains stem cells and differentiated cells.

FIG. 19 shows micrographs of human large intestine-derived organoids on day 7 of culture, which had been established in the absence of Matrigel (registered trademark) and in the presence of Lats-IN and had undergone 18 passages. The scale bar in the left photograph of FIG. 19 is 500 µm, and the scale bar in the right photograph of FIG. 19 is 100 µm.

### [Experimental Example 14]

### (Examination 4 of organoid morphology)

Human large intestine-derived organoids established in the absence of Matrigel (registered trademark) and in the presence of Lats-IN (final concentration 10: µM) were cultured for the first time in the presence of Matrigel (registered trademark) in the same manner as in Experimental Example 7, and their morphology and properties were observed. This experimental example differs from Experimental Example 9 in that the organoid establishment was performed in a Matrigel (registered trademark)-free manner.

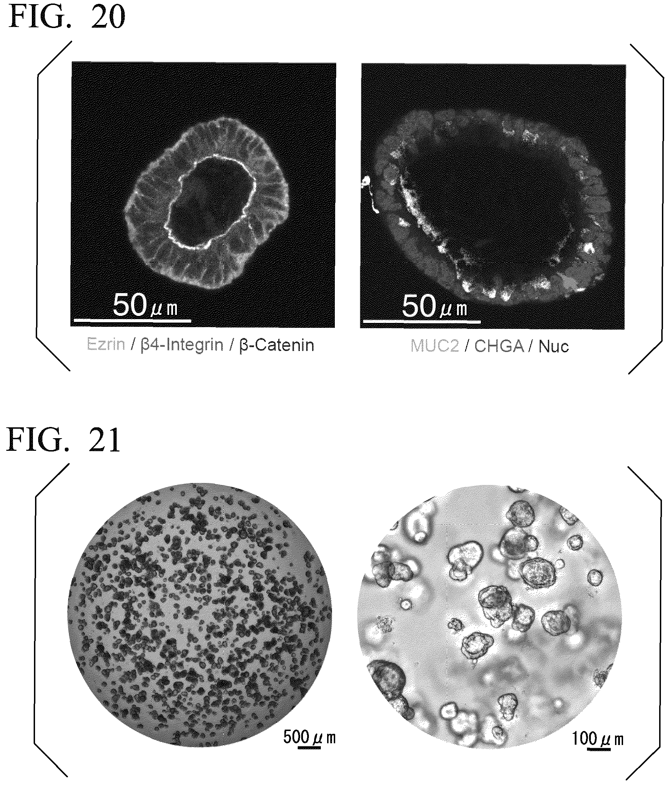

FIG. 20 shows representative photographs of immunostained human large intestine-derived organoids cultured for 7 days in the presence of Matrigel (registered trademark) for the first time, following their establishment in the absence of Matrigel (registered trademark) and in the presence of Lats-IN, and after undergoing 18 passages. In FIG. 20, the scale bar represents 50 µm.

Immunostaining detected the apical marker ezrin, the basement membrane marker integrin β4 (β4-Integrin), the cell-cell adhesion marker β-catenin (β-Catenin), the embryonic cell marker mucin 2 (MUC2), the enteroendocrine cell marker chromogranin A (CHGA), and the nucleus (Nuc).

As a result, as shown in FIG. 20, even the human lower gastrointestinal organoids established in the absence of Matrigel (registered trademark) and in the presence of Lats-IN, when subsequently cultured in the presence of Matrigel (registered trademark), formed a spherical shape in which a monolayer epithelial cell sheet with apical polarity is oriented with the apical side facing inward, and it was revealed that each organoid contained differentiated cells such as embryonic cells and enteroendocrine cells.

This result indicated that the properties of the human lower gastrointestinal epithelial cells were maintained even after the establishment in the absence of Matrigel (registered trademark).

FIG. 21 shows micrographs of human large intestine-derived organoids on day 7 of culture in the presence of Matrigel (registered trademark) for the first time, following their establishment in the absence of Matrigel (registered trademark) and in the presence of Lats-IN, and after undergoing 18 passages. The scale bar in the left photograph of FIG. 21 is 500 µm, and the scale bar in the right photograph of FIG. 21 is 100 µm.

### [Experimental Example 15]

### (Culturing liver cell organoids in absence of Matrigel (registered trademark))

Human liver cell organoids, established from primary human liver cells, were supplemented with TrypLE Express (Thermo Fisher Scientific) and incubated at 37°C for 5 minutes to dissociate them into single cells.

The cells were then collected and suspended in proliferation medium separately supplemented with 2, 5, 10, 20, or 50 µM TDI-011536 (CAS No. 2687970-96-1), and seeded into an ultra-low attachment 96-well plate at 2,000 cells/well. The preferred seeding density is 2,000 to 4,000 cells/well. In addition, cells in proliferation medium supplemented with no TDI-011536 were prepared as a control.

The proliferation medium was a medium consisting of a basal medium (Advanced DMEM/F-12 medium, 10 mM HEPES, 2 mM GlutaMAX, 100 U penicillin, 100 µg/mL streptomycin) supplemented with 10% afamin-Wnt-3A conditioned medium, 2% R-spondin 1 conditioned medium, 2% Noggin conditioned medium, and 20 ng/mL Oncostatin M.

On day 6 after seeding, the number of viable cells in the human liver cell organoids was measured using a commercially available kit (product name "CellTiter-Glo 3D," Promega Corporation).

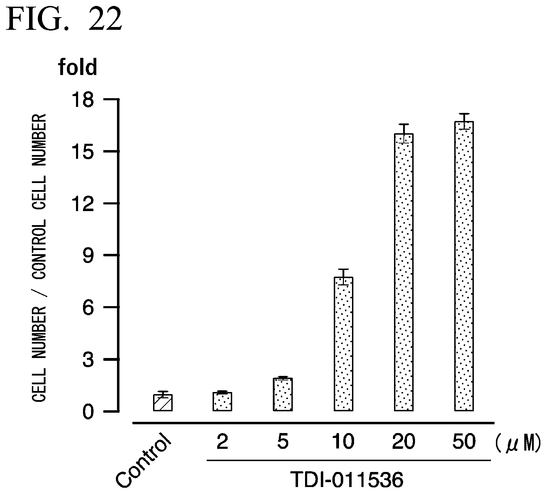

FIG. 22 is a graph showing the results of measuring the number of viable cells. As a result, in the groups supplemented with 20 and 50 µM TDI-011536 as LATS-IN, the number of viable cells was measured to be 16-fold and 17-fold higher than that in the control group. The increase in the number of viable cells was also observed in other LATS-INs, such as TRULI, where the number of viable cells increased by about three-fold, which revealed that an increase in the number of viable cells in the human liver cell organoids was observed in the absence of Matrigel and in the presence of LATS-IN.

### [Experimental Example 16]

### (Establishment of liver cell organoids in absence of Matrigel (registered trademark))

Frozen primary human liver cells were suspended in Advanced DMEM/F-12 medium and thawed. Two types of primary human liver cells, Line 1 and Line 2, were used.

Subsequently, each of the thawed primary human liver cells was stained with an APC-labeled mouse anti-human EpCAM antibody, and EpCAM-negative cells were collected using a cell sorter. The collected cells were then suspended in proliferation medium supplemented with 20 µM TDI-011536 and seeded into an ultra-low attachment 96-well plate at 3,000 to 4,000 cells/well. The cells were then cultured to obtain liver cell organoids. In addition, for comparison, cells were also prepared using proliferation medium supplemented with no TDI-011536 or no Oncostatin M.

The passaging of liver cell organoids in the absence of Matrigel (registered trademark) was performed as follows. First, calcium ions and magnesium ions were chelated using 1 mM EDTA/phosphate buffered saline (PBS). Subsequently, the liver cell organoids were supplemented with TrypLE Express (Thermo Fisher Scientific) and incubated at 37°C for 5 minutes to dissociate them into single cells.

The collected cells were then suspended in proliferation medium supplemented with 20 µM TDI-011536 and seeded into an ultra-low attachment 96-well plate at 2,000 cells/well.

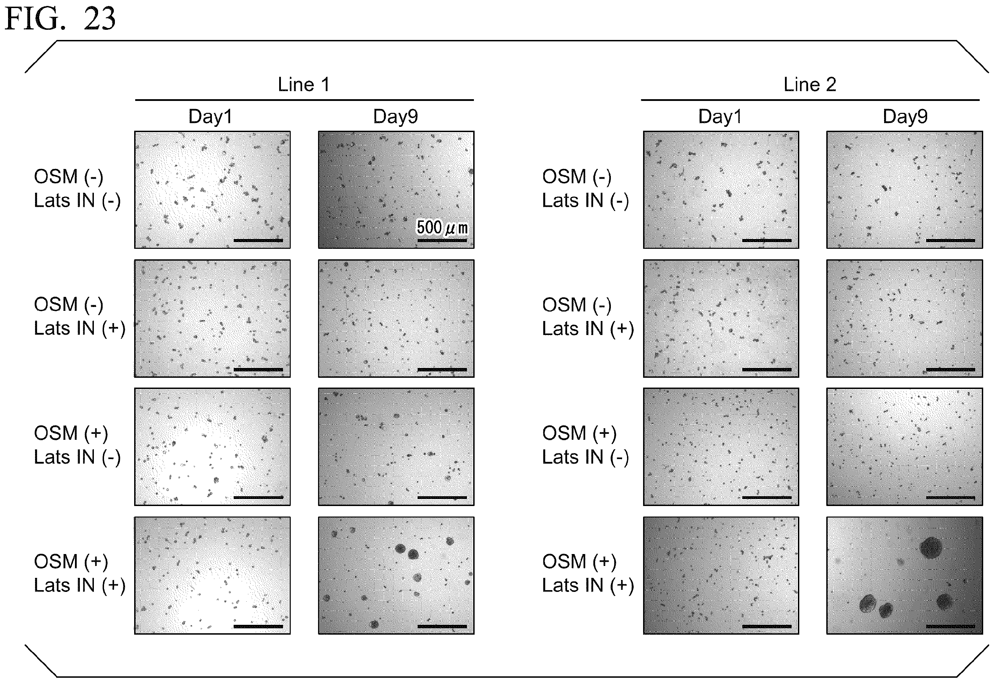

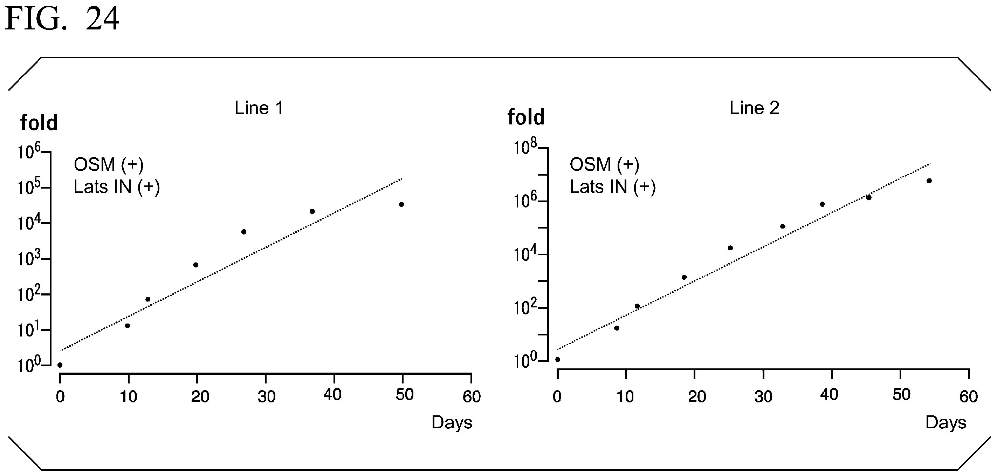

FIG. 23 shows micrographs of the cells taken 1 day and 9 days after the start of culture. The scale bar is 500 µm. FIG. 24 presents graphs showing the results of measuring changes in cell proliferation over time. In FIG. 24, the vertical axes of the graphs represent cell proliferation (fold increase), and the horizontal axes indicate the number of days of culture. In FIGS. 23 and 24, "OSM" represents Oncostatin M, and "Lats IN" represents the LATS kinase inhibitor (TDI-011536).

As a result, as shown in FIG. 22, an increase in the number of viable cells was observed by adding the LATS kinase inhibitor (TDI-011536), and further, as shown in FIG. 23, when proliferation medium supplemented with both the LATS kinase inhibitor (TDI-011536) and Oncostatin M was used, favorable human liver cell organoids were successfully established. The established human liver cell organoids could be cultured for at least two months, and the cell number increased by 10⁶ to 10⁸-fold from establishment (FIG. 24).

### [Experimental Example 17]

### (Examination of liver cell organoid cultured in absence of Matrigel (registered trademark))

A liver cell organoid cultured in the absence of Matrigel (registered trademark) was subjected to fluorescent immunostaining to confirm that it was a liver cell. For comparison, fluorescent immunostaining of a liver cell organoid cultured in the presence of Matrigel (registered trademark) was also performed.

First, human liver cell organoids were fixed using 1% formaldehyde/HEPES buffered saline (HBS). Subsequently, the fixed human liver cell organoids were then perforated with 1% Triton X-100/PBS. Subsequently, the human liver cell organoids were blocked with 1% BSA/PBS. Subsequently, HNF4α, a hepatocyte marker, was immunostained using mouse anti-HNF4α antibody (Thermo Fisher Scientific Inc.).

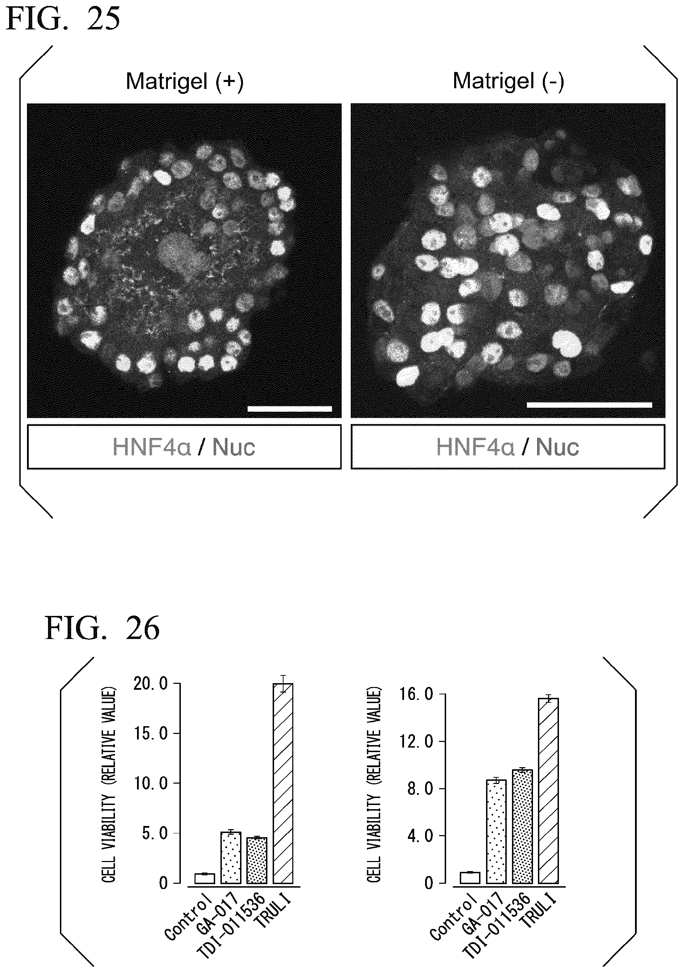

FIG. 25 presents micrographs showing the results of fluorescent immunostaining. The scale bar is 50 µm. In FIG. 25, "Matrigel (+)" shows the result for the liver cell organoid cultured in the presence of Matrigel (registered trademark), and "Matrigel(-)" shows the result for the liver cell organoid cultured in the absence of Matrigel (registered trademark). In addition, "Nuc" shows the result of staining the nucleus.

The results confirmed that the human liver cell organoid cultured in the absence of Matrigel (registered trademark) also expressed HNF4α, a hepatocyte marker, similarly to the liver cell organoid cultured in the presence of Matrigel (registered trademark). These results indicate that the human liver cell organoid cultured in the absence of Matrigel (registered trademark) is also definitely a liver cell organoid.

### [Experimental Example 18]

### (Culture 3 of organoids in presence of LATS kinase inhibitor)

In this experimental example, human small intestine-derived organoids and human large intestine-derived organoids were cultured in the absence of Matrigel (registered trademark) and in the presence of each LATS kinase inhibitor (final concentration: 20 µM) or in the absence of a LATS kinase inhibitor (control) in the same manner as in Experimental Example 2, and their dynamics were observed.

As LATS kinase inhibitors, GA-017 (CAS No. 2351906-74-4), TDI-011536 (CAS No. 2687970-96-1), and TRULI (CAS No. 1424635-83-5, also called "Lats-IN" or "Lats-IN-1") were used.

FIG. 26 presents graphs showing the results of measuring cell number in each organoid after 6 days of culture by measuring ATP using a commercially available kit (product name "CellTiter-Glo 3D," Promega Corporation). The vertical axis of the graph represents luminescence intensity (relative value) indicating the amount of ATP present. The left side of FIG. 26 shows the results for human large intestine-derived organoids, and the right side of FIG. 26 shows the results for human small intestine-derived organoids.

As a result, it was revealed that, regardless of which LATS kinase inhibitor was used, cell proliferation was significantly activated in both the human small intestine-derived and human large intestine-derived organoids even in the absence of Matrigel (registered trademark).

### [Experimental Example 19]

### (Examination 2 of organoid formation from single cells)

After dissociating human large intestine-derived organoids into single cells, organoid formation from the single cells was observed.

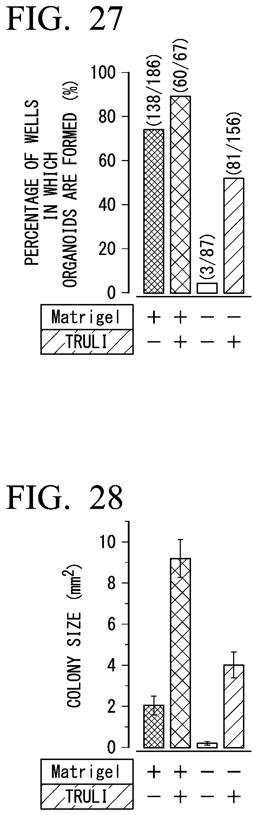

FIG. 27 is a graph showing the results of organoid formation from the single cells in the presence (+) or absence (-) of Matrigel (registered trademark) and in the absence (-) or presence (+, final concentration: 10 µM) of TRULI. In the parentheses of FIG. 27, the denominator shows the number of single cells seeded, and the numerator shows the number of organoids formed. In addition, the vertical axis of the graph represents the percentage (%) of cells that formed organoids among the seeded single cells.

The results showed that cells hardly proliferated in the absence of Matrigel (registered trademark) and TRULI. In contrast, it was revealed that cells proliferated from single cells and formed organoids in the presence of Matrigel (registered trademark), and in the absence of Matrigel (registered trademark) and in the presence of TRULI.

### [Experimental Example 20]

### (Examination of colony size)

After dissociating human small intestine-derived organoids into single cells, organoid formation from the single cells was observed. FIG. 28 is a graph showing the results of measuring the colony size after forming organoids from the single cells in the presence (+) or absence (-) of Matrigel (registered trademark) and in the absence (-) or presence (+, final concentration: 10 µM) of TRULI. The colony size was measured using software (lmageJ). In FIG. 28, the vertical axis represents the colony size (mm²).

### [Experimental Example 21]

### (Culture 4 of organoids in presence of LATS kinase inhibitor)

Organoids derived from various human tissues were dissociated into single cells, and then cultured in the absence of Matrigel (registered trademark) and in the presence of each LATS kinase inhibitor, or in the absence of Matrigel (registered trademark) and a LATS kinase inhibitor (control) in the same manner as in Experimental Example 2, and their dynamics were observed.

As LATS kinase inhibitors, TDI-011536 (CAS No. 2687970-96-1) and TRULI (CAS No. 1424635-83-5, also called "Lats-IN" or "Lats-IN-1") were used. The concentration of the LATS kinase inhibitor varied depending on the organoid and was set between 5 and 20 µM.

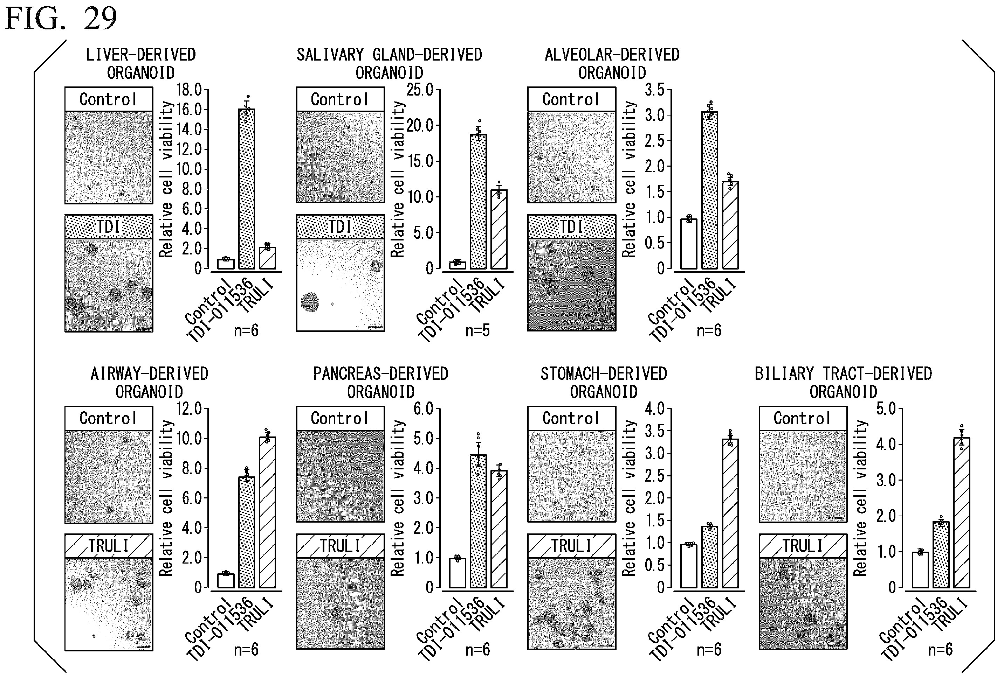

FIG. 29 presents bright-field micrographs of organoids after 6 days of culture, and graphs showing the results of measuring cell number in each organoid by measuring ATP using a commercially available kit (product name "CellTiter-Glo 3D," Promega Corporation). The vertical axes of the graphs represent luminescence intensity (relative value) indicating the amount of ATP present. In FIG. 29, the upper left panel shows the results for human liver-derived organoids, the upper center panel shows the results for human salivary gland-derived organoids, the upper right panel shows the results for human alveolar-derived organoids, the lower left panel shows the results for human airway-derived organoids, the second panel from the left in the lower row shows the results for human pancreas-derived organoids, the third panel from the left in the lower row shows the results for human stomach-derived organoids, and the lower right panel shows the results for human biliary tract-derived organoids. In addition, "Control" shows the result for a control, and "TDI" shows the result for a group to which TDI-011536 was added.

As a result, it was revealed that, regardless of which LATS kinase inhibitor was used, cell proliferation of the organoids derived from the various tissues was significantly activated in the absence of Matrigel (registered trademark).

### [Experimental Example 22]

### (Organoid establishment in absence of Matrigel (registered trademark))

Crypts were collected from the human colon and dissociated into single cells, then cultured in the presence of Matrigel (registered trademark) or in the absence of Matrigel (registered trademark) and in the presence of TRULI to establish organoids. In addition, the cell number in each organoid under the given conditions was measured by measuring ATP using a commercially available kit (product name "CellTiter-Glo 3D," Promega Corporation).

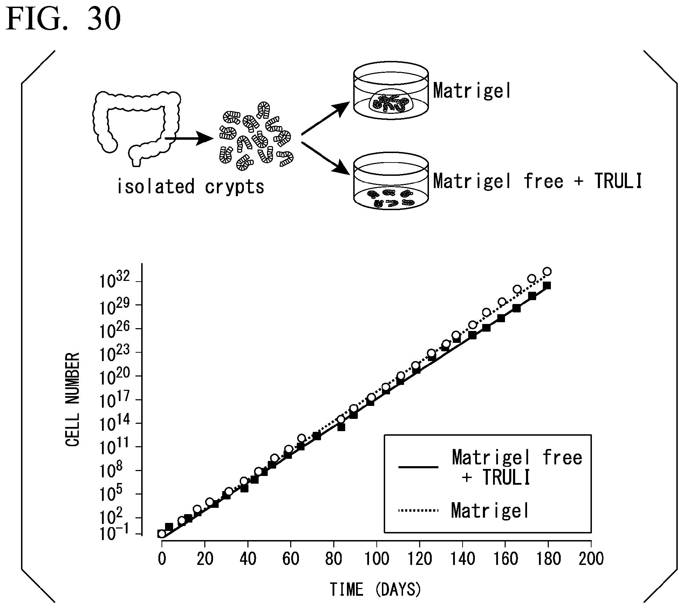

The upper panel of FIG. 30 is a schematic view explaining an overview of this experimental example. The lower panel of FIG. 30 is a graph showing the results of measuring the proliferation of the organoids. As a result, in the presence of TRULI, cell proliferation comparable to that observed in the presence of Matrigel (registered trademark) was observed even in the absence of Matrigel (registered trademark).

### [Experimental Example 23]

### (Orthotopic xenotransplantation of organoids established in absence of Matrigel (registered trademark))

The human colon-derived organoids established in the absence of Matrigel (registered trademark) and in the presence of TRULI in Experimental Example 22 were orthotopically xenotransplanted into an immunodeficient mouse (NOG mouse) to examine whether they would engraft.

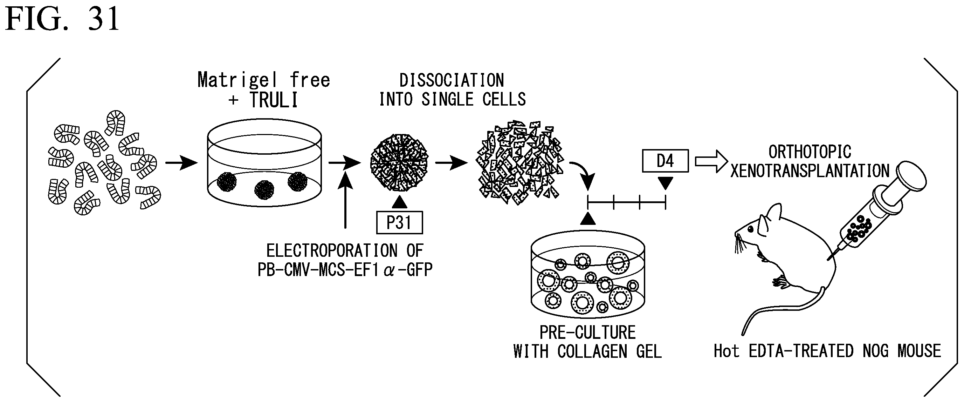

FIG. 31 is a schematic view explaining an overview of this experimental example. The human colon-derived organoids established in the absence of Matrigel (registered trademark) and in the presence of TRULI were electroporated with a green fluorescent protein (GFP) expression vector to obtain GFP-labeled human colon-derived organoids.

Subsequently, the GFP-labeled human colon-derived organoids were dissociated into single cells and then pre-cultured for 4 days in a medium supplemented with 2% Matrigel (registered trademark) or 2% collagen gel (50% type IA, 50% type IC). For 2% collagen gel culture, the medium was supplemented with 2.5 µM 16,16-dimethyl prostaglandin E2 (dmPGE2, Cayman Chemical Company), 50 ng/mL human recombinant hepatocyte growth factor (HGF, PeproTech Inc.), 20 ng/mL human recombinant oncostatin M (OSM, PeproTech Inc.), and 5 nM human recombinant heregulin β1 (NRG1, PeproTech Inc.).

Subsequently, the colonic epithelium and mucosa of the NOG mouse were removed and orthotopically xenotransplanted with the GFP-labeled human colon-derived organoids. First, the NOG mouse fed a normal diet was anesthetized by inhalation of 2-3% isoflurane. Subsequently, the colon of the mouse was washed with PBS to remove luminal contents. A catheter equipped with a small balloon was then inserted into the anus and the balloon was inflated with air to hold the catheter. Subsequently, hot EDTA (250 mM, 50 to 55°C) was injected into the rectum to remove the colonic mucosa and chelate Ca²⁺ and Mg²⁺, which are necessary for cell-cell adhesion and cell-extracellular matrix adhesion.

Subsequently, the colonic epithelium and mucosa were scrubbed using a vibrating electric toothbrush (EW-DL22, EW0945, Panasonic Corporation). Successful removal of the epithelium and mucosa was confirmed by releasing the isolated colonic epithelium from the vibrating electric toothbrush into a water-filled Petri dish.

Subsequently, the total cell number of the pre-cultured GFP-labeled human colon-derived organoids was counted, and the cells were suspended in Advanced DMEM/F12 medium supplemented with 10% (vol/vol) Matrigel (registered trademark) or type IA collagen. Using a 200 µL pipette, 70 µL of the organoid suspension containing 1 × 10⁷ cells was injected into the colon of the NOG mouse (recipient mouse) from which the colonic epithelium and mucosa had been removed. Subsequently, the anus of the mouse was temporarily sealed with soft paper using adhesive to maintain the engraftment of the GFP-labeled human colon-derived organoids in the colon. To examine the occurrence of intestinal obstruction, feces of the recipient mouse were carefully observed for one week.

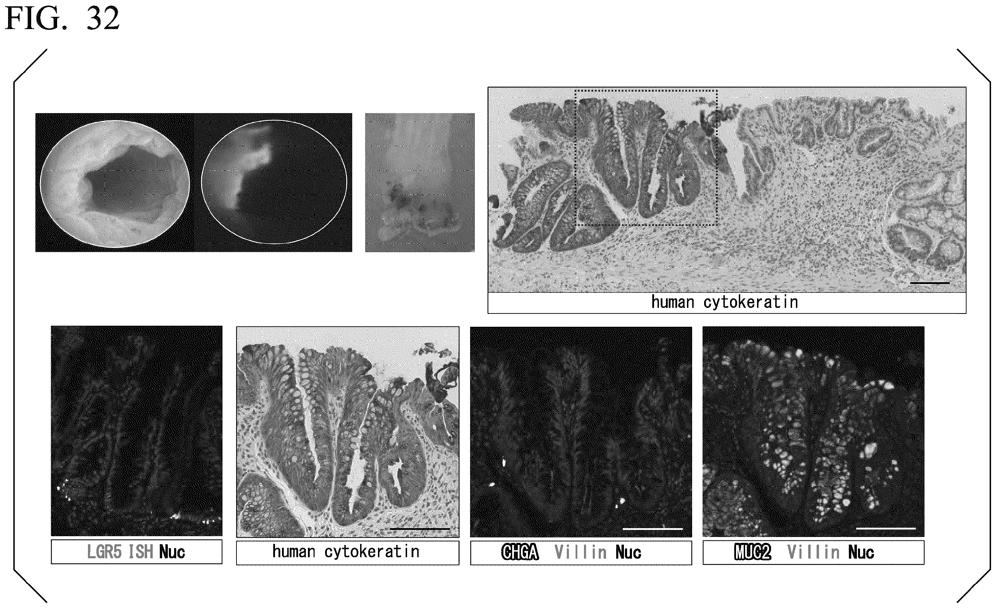

The leftmost panel in the upper row of FIG. 32 is a bright-field photograph observing the colon of the NOG mouse transplanted with the GFP-labeled human colon-derived organoids. The second panel from the left in the upper row of FIG. 32 is a photograph of GFP fluorescence taken in the same field of view as the leftmost panel in the upper row of FIG. 32. The third panel from the left in the upper row of FIG. 32 is a photograph of the intestine of the mouse with a damaged area stained. The upper right panel of FIG. 32 is a micrograph showing the result of immunostaining a colon section of the NOG mouse transplanted with the GFP-labeled human colon-derived organoids using an anti-human cytokeratin antibody. The scale bar is 200 µm.

The leftmost panel in the lower row of FIG. 32 is a fluorescent micrograph showing the result of staining the area surrounded by the dotted line in the upper right panel of FIG. 32 by in situ hybridization for the mRNA of the human LGR5 gene. The second panel from the left in the lower row of FIG. 32 is an enlarged photograph of the area surrounded by the dotted line in the upper right panel of FIG. 32. The scale bar is 100 µm. The third panel from the left in the lower row of FIG. 32 is a fluorescence micrograph showing the result of staining the area surrounded by the dotted line in the upper right panel of FIG. 32 with an anti-human chromogranin A (CHGA) antibody and an anti-human villin antibody. The scale bar is 100 µm. The lower right panel of FIG. 32 is a fluorescence micrograph showing the result of staining the area surrounded by the dotted line in the upper right panel of FIG. 32 with an anti-human mucin 2 (MUC2) antibody and an anti-human villin antibody. The scale bar is 100 µm.

From the above results, the human colon-derived organoids established in the absence of Matrigel (registered trademark) and in the presence of TRULI could engraft through orthotopic xenotransplantation into the immunodeficient mouse (NOG mouse). This revealed that organoids produced in the absence of Matrigel and in the presence of TRUL1 can be used in regenerative medicine.

### INDUSTRIAL APPLICABILITY

According to the present invention, it is possible to provide technology for culturing an organoid in the absence of an extracellular matrix.

## Claims

1. An agent for organoid formation and proliferation in the absence of an extracellular matrix, the agent comprising:
a Hippo signaling pathway inhibitor as an active component.

2. The agent for organoid formation and proliferation in the absence of an extracellular matrix according to claim 1,
wherein the Hippo signaling pathway inhibitor is an MST1/2 kinase inhibitor or a large tumor suppressor kinase (LATS) 1/2 kinase inhibitor.

3. The agent for organoid formation and proliferation in the absence of an extracellular matrix according to claim 1 or 2, the agent further comprising:
a cytokine family that binds to gp130.

4. The agent for organoid formation and proliferation in the absence of an extracellular matrix according to claim 1 or 2,
wherein the organoid is an epithelial organoid.

5. A method for organoid proliferation, comprising:
a step of culturing an organoid in the presence of a Hippo signaling pathway inhibitor and in the absence of an extracellular matrix.

6. The method for organoid proliferation according to claim 5,
wherein the organoid is cultured under serum-free conditions.

7. An organoid proliferated by the proliferation method according to claim 5 or 6.

8. An agent for establishing an organoid in the absence of an extracellular matrix, the agent comprising:
a Hippo signaling pathway inhibitor as an active component.

9. The agent for establishing an organoid in the absence of an extracellular matrix according to claim 8,
wherein the Hippo signaling pathway inhibitor is an MST1/2 kinase inhibitor or a LATS1/2 kinase inhibitor.

10. The agent for establishing an organoid in the absence of an extracellular matrix according to claim 8 or 9, the agent further comprising:
a cytokine family that binds to gp130.

11. The agent for establishing an organoid in the absence of an extracellular matrix according to claim 8 or 9,
wherein the organoid is an epithelial organoid.

12. A method for producing an organoid, comprising:
a step of establishing an organoid in the presence of a Hippo signaling pathway inhibitor and in the absence of an extracellular matrix.

13. The method for producing an organoid according to claim 12,
wherein the organoid is established in the presence of a cytokine family that binds to gp130.

14. The method for producing an organoid according to claim 12 or 13,
wherein the organoid is established under serum-free conditions.

15. An organoid produced by the production method according to claim 12 or 13.

16. A regenerative medicine preparation comprising:
the organoid according to claim 15 as an active component.

17. A method for screening for an agent that enables culture of an organoid in the absence of an extracellular matrix, the method comprising:
a step of culturing an organoid in the presence of a test substance and in the absence of an extracellular matrix; and
a step of evaluating proliferation of the organoid,
wherein an increase in the proliferation of the organoid compared to when the test substance is absent indicates that the test substance is an agent that enables organoid culture in the absence of an extracellular matrix.
